# EUROPÄISCHE PATENTANMELDUNG

(11) **EP 3 556 731 A1**
(43) Veröffentlichungstag der Anmeldung: **23.10.2019**
(21) Anmeldenummer: 18168122.2
(22) Anmeldetag: 19.04.2018
(51) Int. Cl.: C01G 23/053, C09C 1/36, C09C 3/12, A61K 9/00, C08K 9/06

(54) **VERFAHREN ZUR CHEMISCHEN MODIFIZIERUNG VON METALLOXIDOBERFLÄCHEN**

(71) Anmelder: Justus-Liebig-Universität Gießen, 35390 Gießen (DE)
(72) Erfinder: Dr. Suchomski, Christian, 35102 Lohra (DE); Prof. Dr. Smarsly, Bernd, 35415 Pohlheim (DE); Hofmann, Anja, 06132 Halle (DE)
(74) Vertreter: Stumpf, Peter

(57) **Zusammenfassung**

Die Erfindung betrifft ein effektives und kostengünstiges Verfahren zur chemischen Modifikation von Metalloxid-Oberflächen, insbesondere Partikeloberflächen und ganz besonders Metalloxid-Nanopartikel-Oberflächen. Das Verfahren umfasst die Vorbehandlung der Metalloxidoberfläche mit einem polar-protischen Lösungsmittel, die Abtrennung der Partikel bzw. Reinigung der MetalloxidOberfläche durch Zugabe eines polar-protischen Fällungs- bzw. Reinigungsmittel sowie die anschließende Durchführung der Oberflächenmodifizierungsreaktion, bevorzugt in Form einer Silanisierung.

## Beschreibung

Die vorliegende Erfindung betrifft ein Verfahren zur Oberflächenmodifikation von Metalloxid-Nanopartikeln, größeren Metalloxidpartikeln sowie makroskopischen Metalloxidoberflächen.

### Stand der Technik

Metalloxidpartikel bzw. Metalloxid-Nanopartikel - beide Begriffe werden im weiteren synonym verwendet - werden oftmals als Füllstoffe in Polymeren eingesetzt, um deren mechanische und/oder optische Eigenschaften zu verbessern. Sie finden damit Verwendung in zahlreichen unterschiedlichen Anwendungsbereichen, beispielsweise in Polymeren für Dentalanwendungen, 3D-Druckverfahren oder Korrosionsschutz-Beschichtungen. Um die Metalloxid-Nanopartikel fest mit der Polymermatrix zu verbinden und/oder ihre Benetzungseigenschaften zu verbessern, werden sie vorteilhafterweise oberflächlich chemisch modifiziert, d.h. es werden organische Verbindungen chemisch an die Partikeloberfläche gebunden, die ihrerseits gut in der gewünschten Polymermatrix löslich sind und/oder Substituenten aufweisen, mit denen sie chemisch an die Polymermatrix gebunden werden können.

Zweckmäßig werden Oberflächenfunktionalisierungen von Metalloxiden mit (organo)funktionellen Silanen unter basischen Bedingungen durchgeführt. Ein Beispiel hierfür ist die Funktionalisierung von Zinkoxid-Nanopartikeln mit 3-(Methoxysilyl)propylmethacrylat bei pH-Werten zwischen 9 und 12 unter Verwendung von Ammoniakwasser (siehe C. Bressy, V. Giang Ngo, F. Ziarelli, A. Margaillan, Langmuir 2012, 28, 3290-3297). Allerdings ist dieses Verfahren nicht für Metalloxide wie zum Beispiel Zirconiumdioxid, Titandioxid u. a. geeignet, da im alkalischen Milieu ein hoher Gehalt an Hydroxid-Ionen zur Präzipitation der Partikel führt und deshalb eine Silanisierung der Partikeloberfläche nicht möglich ist.

Um dieses Problem zu vermeiden, können alternativ Silanisierungen unter sauren Bedingungen durchgeführt werden. Ein Beispiel hierfür ist die Verwendung von organischen Säuren wie Maleinsäureanhydrid (siehe Publikation F. Bauer, H. Ernst, U. Decker, M. Findeisen, H.-J. Gläsel, H. Langguth, E. Hartmann, R. Mehnert, C. Peuker, Macromol. Chem. Phys. 2000, 201, 2654-2659). Die Reaktionsführung, z.B. Einstellung von Temperatur, Rührgeschwindigkeit und pH-Wert, ist von zentraler Bedeutung, da Nebenreaktionen zur Bildung von Core-Shell-Strukturen führen können (vgl. Anmeldung US 20100178512 A1).

Es existiert eine Vielzahl an Verfahren für die Oberflächenmodifizierung von SiO₂-Nanopartikeln. Diese sind aber nur begrenzt auf andere Metalloxide übertragbar und es ist im Stand der Technik notwendig, die Metalloxide mit Basen oder Säuren vorzubehandeln, bzw. unter Säure- oder Basenkatalyse zu arbeiten, um eine einigermaßen zufriedenstellende Silanisierung zu erzielen. Diese Säure- oder Basenbehandlung führt jedoch auch zur Partikelagglomeration, so dass die anschließende oberflächliche Silanisierungsreaktion nur sehr asymmetrisch erfolgt. Es verbleiben Bereiche auf der Oberfläche der Partikel, die nicht silanisiert werden. Zudem bilden sich bei der weiteren Verarbeitung der so silanisierten Partikel zusätzliche nicht-silanisierte Bereiche, wenn die Agglomerate nachfolgend absichtlich oder unabsichtlich wieder aufgebrochen werden, beispielsweise durch nachfolgende Dispergierprozesse. Zudem sind Formulierungen, Zubereitungen aus derartig silanisierten Partikeln aufgrund der Partikel-Agglomeratbildung für gewöhnlich nicht langzeitstabil.

Im Stand der Technik wird somit nur eine ungleichmäßige Modifizierung bzw. Silanisierung der Oberfläche von Metalloxidpartikeln erreicht und im Rahmen der nachfolgenden Verarbeitung wird diese zwangsläufige Ungleichmäßigkeit noch vergrößert, wenn die oberflächlich modifizierten Partikelaggregate beabsichtigt oder unbeabsichtigt wieder aufgebrochen werden.

### Aufgabe

Aufgabe der vorliegenden Erfindung ist es, ein gegenüber dem Stand der Technik einfaches und somit kostengünstigeres Verfahren zur Silanisierung von Metalloxidpartikeln bereitzustellen, das ohne die Verwendung von Säuren oder Laugen auskommt und die im Stand der Technik bestehenden Nachteile der ungleichmäßigen Modifizierung der Partikeloberflächen vermeidet.

### Lösung der Aufgabe

Diese Aufgabe wird erfindungsgemäß gelöst durch die kennzeichnenden Merkmale des Hauptanspruchs. Die Unteransprüche stellen weitere vorteilhafte Ausgestaltungen der Erfindung dar.

Die Erfindung wird nachfolgend am Beispiel der Silanisierung von Metalloxid-Nanopartikeln offenbart. Es ist jedoch für den Fachmann ohne weiteres erkennbar, dass die kennzeichnenden Merkmale der Erfindung, beispielsweise die vorbereitende Konditionierung/Reinigung der Oberfläche der Nanopartikel für die eigentliche Silanisierungsreaktion, nicht auf die Oberfläche von Metalloxid-Nanopartikeln beschränkt ist, sondern auch für die Oberflächen größerer Metalloxidpartikel und sogar für makroskopische Metalloxid-Oberflächen gilt. Wo im folgenden von Metalloxid-Nanopartikeln die Rede ist, sind daher immer auch größere Metalloxidpartikel sowie auch makroskopische Metalloxid-Oberflächen mit umfasst. Gleichermaßen umfassen die Nanopartikel-spezifischen Verfahrensschritte immer auch die analogen Verfahrensschritte für größere Partikel und makroskopische Oberflächen. Beispielsweise ist, wann immer von "Nanopartikel-Dispersionen", "Dispersion" bzw. "dispergieren" die Rede ist, implizit auch von "Partikelsuspensionen", "Suspension", bzw. "suspendieren" die Rede; bezüglich makroskopischer Oberflächen ist dann analog die mit Lösungsmittel benetzte Oberfläche gemeint. Wenn von "Aufarbeitung", "Trennung", "Überführung" und dergleichen die Rede ist, sind damit immer auch in analoger Weise entsprechende Reinigungs- bzw. Behandlungsschritte an makroskopischen Oberflächen gemeint, beispielsweise das Abspülen der Oberfläche mit dem betreffenden Lösungsmittel, oder auch sowohl das Eintauchen als auch das Besprühen mit der jeweiligen Lösung.

Die Vorteilhaftigkeit der Erfindung über Nanopartikel und Partikel hinaus bis hin zu makroskopischen Oberflächen ergibt sich direkt aus den Vorteilen im Bereich der Nanopartikel. Diese können beispielsweise durch die äußerst gleichmäßige und vollständige Silanisierung mit einem Silanisierungsmittel, das über mindestens einen polymerisierbaren Substituenten verfügt, chemisch direkt an entsprechende Polymere angebunden werden und weisen damit eine besonders gute Haftung und optimale Verteilung im daraus resultierenden Polymermaterial auf. Übertragen auf die Situation einer makroskopischen Metalloxidoberfläche, beispielhaft sei ein Keramikbauteil oder ein, eine entsprechende Oxidschicht aufweisendes, Metallteil genannt, etwa aus eloxiertem Aluminium, bedeutet das, dass die erfindungsgemäße Silanisierung des Bauteils mit einem entsprechenden Silanisierungsmittel dazu führt, dass dieses besonders gut haftend in eine Polymermatrix einpolymerisiert werden kann, da ein fester chemischer Verbund von Metall-/Keramik-Bauteil und fertigem Polymerbauteil erhalten wird.

Als Silanisierung wird gemeinhin die chemische Anbindung einer Silanverbindung an eine Oberfläche bezeichnet. Die Anbindung erfolgt im Allgemeinen durch Kondensationsreaktionen zwischen hydrolysierbaren Gruppen der verwendeten Silane und chemischen Gruppen an der Oberfläche, hier beispielhaft der Nanopartikel oberfläche. Bekannterweise erfolgt die Anknüpfung der Silane bzw. Silanisierungsmittel - beide Begriffe werden im weiteren synonym verwendet - über die freien OH-Gruppen auf der Nanopartikeloberfläche. SiO₂-Partikel verfügen über besonders viele freie OH-Gruppen, zahlreiche Metalloxide, etwa Zircondioxid (ZrO₂), Titandioxid (TiO₂), Aluminiumoxid (Al₂O₃) u.a., jedoch über deutlich weniger. Daraus folgt zwangsläufig die schwierigere und unvollständigere Silanisierung dieser Metalloxid-Nanopartikel. Erfindungsgemäß wird die Anzahl der freien OH-Gruppen auf den nano-, mikro- oder makroskopischen Metalloxidoberflächen durch Vorbehandlung dieser mit Wasser oder einem anderen polar-protischen Lösungsmittel erreicht, indem die Metalloxidoberfläche in intensiven direkten Kontakt mit dem polar-protischen Lösungsmittel gebracht wird. Dies erfolgt erfindungsgemäß sowohl bei Umgebungstemperatur/Raumtemperatur als auch bei erhöhter Temperatur oder gar unter überkritischen Bedingungen bezüglich des jeweiligen Lösungsmittels. Unter dem Begriff "Lösungsmittel" sind im Rahmen dieser Offenbarung sowohl echte Lösungsmittel gemeint, die einen Stoff auflösen können, als auch jegliche Arten von Flüssigkeiten, in denen Metalloxid-Nanopartikel dispergiert bzw. Metalloxidpartikel suspendiert/aufgeschlämmt oder mit denen Metalloxidoberflächen benetzt werden können. Das heißt, ein Lösungsmittel im Sinne der vorliegenden Erfindung muss nicht zwingend etwas auflösen, es wird jedoch in jedem Fall in der Lage sein, eine Metalloxidoberfläche zu benetzen. Insbesondere kann ein erfindungsgemäßes Lösungsmittel natürlich auch beides parallel, also eine Metalloxidoberfläche benetzen (Nanopartikel dispergieren, Partikel suspendieren) und gleichzeitig andere Substanzen lösen, etwa Silanisierungsmittel, Silanisierungshilfsmittel, weitere Edukte für die Silanisierungsreaktion oder ggf. Katalysatoren. Der Begriff "Lösung" umfasst daher beispielsweise auch Dispersionen und Suspensionen. Neben Reinsubstanzen umfasst der Begriff "Lösungsmittel" implizit auch Mischungen von zwei oder mehr Reinsubstanzen, die im jeweiligen Mischungsverhältnis und bei der jeweiligen Bearbeitungstemperatur eine flüssige Phase geeigneter Polarität und Mischbarkeit bilden, um die Metalloxidoberfläche zu benetzen und somit Partikel zu suspendieren und Nanopartikel zu dispergieren. Das vorstehend über Lösungsmittel gesagte gilt analog auch für die Fällungsmittel, die als Reinsubstanz oder Mischung mit geeigneter Polarität die Ausfällung der Metalloxid-Nanopartikel bewirken.

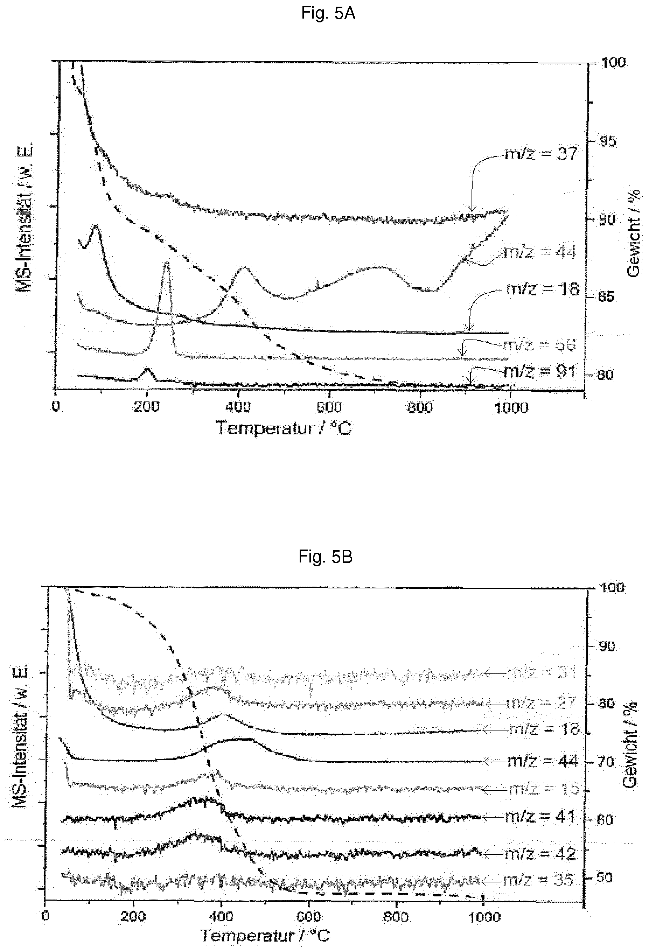

Die Vorbehandlung mit einem polar-protischen Lösungsmittel, wobei Wasser besonders bevorzugt ist, bewirkt beispielsweise die Entstehung zusätzlicher OH-Gruppen auf der Metalloxidoberfläche, indem herstellungsbedingt auf der Oberfläche verbliebene hydrolysierbare Oberflächenliganden, etwa Halogenide oder Alkoholatgruppen, hydrolysiert werden. Die Vorbehandlung bewirkt somit eine Reinigung der Oberfläche auf molekularer Ebene, indem an der Oberfläche gebundene Liganden (die prinzipiell vorhandene Hydroxylgruppen blockieren) entfernt werden. Belegt wird dieser Effekt beispielsweise durch die Ergebnisse der massenspektroskopischen Untersuchungen an silanisierten und nicht silanisierten Proben, vgl. beispielsweise Fig. 5A gegenüber Fig. 5B-E: Das beispielhafte nicht-silanisierte ZrO₂-Nanopulver (Fig. 5A) trägt nachweislich C₆- und C₇-Reste auf der Oberfläche, die sich im Massenspektrum durch entsprechende Signale charakteristischer Fragment-Ionen verraten, die bei den erfindungsgemäß silanisierten Nanopartikeln nicht mehr vorkommen. Eine sehr gute Dispergierbarkeit ist dabei im Falle von Metalloxid-Nanopartikeln (und zwar bei diesen ganz besonders!) vorteilhaft, da die dadurch bereits vorliegenden Primärpartikel allseitig gleichmäßig hydroxyliert werden. Die wie weiter unten beschrieben durchgeführte eigentliche Silanisierungsreaktion kann somit ganzflächig direkt an der Oberfläche der dispergierten Primärpartikel stattfinden, und ein nachfolgendes beabsichtigtes oder unbeabsichtigtes Aufbrechen (teil-)silanisierter Partikel-Agglomerate, das neue, nicht-silanisierte Oberflächen erzeugen würde, kann nicht auftreten. Das Gesagte gilt natürlich analog im Falle größerer Partikel im Mikrometerbereich, sowie - wenn auch deutlich abgeschwächt - für makroskopische Metalloxidoberflächen. Grundsätzlich kann dieser Verfahrensschritt durch den Einsatz von Ultraschall weiter verbessert werden, ohne den Umfang der Erfindung oder ihrer Äquivalente zu verlassen, da die Beschallung mit Ultraschall grundsätzlich dispergierende Wirkung auf Partikel ausübt, sowie reinigende Wirkung auf makroskopische Oberflächen. Auch der Einsatz von Mikrowellen ist möglich, beispielsweise zur Erhöhung der Temperatur.

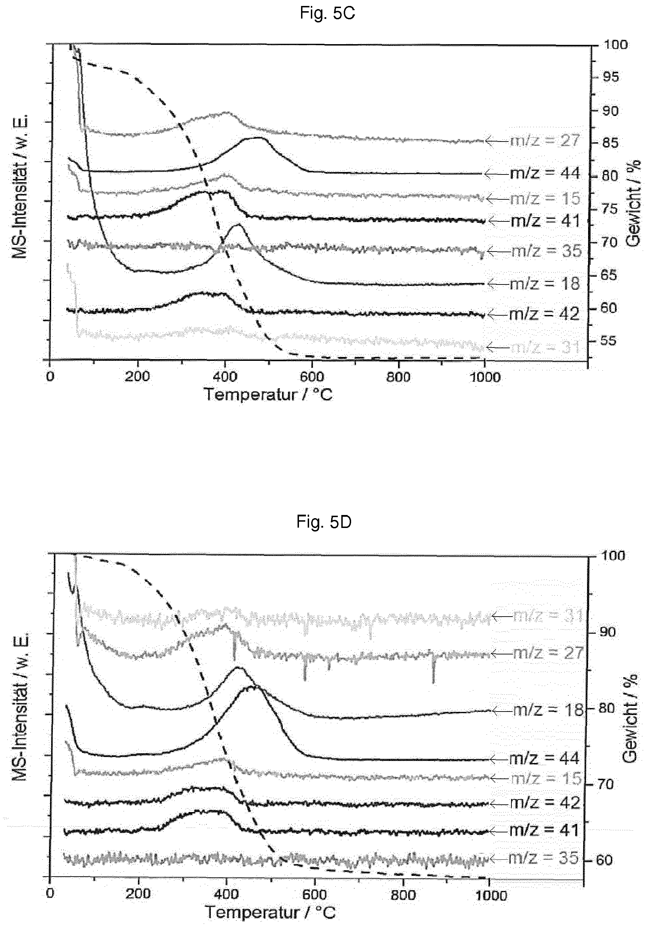

Überraschenderweise werden die positiven Effekte für eine nachfolgende Funktionalisierung nicht nur erreicht, wenn - wie vorstehend beschrieben - oberflächengebundene OH-Gruppen vollständig "entschützt" werden, sondern auch dann, wenn die Vorbehandlung mit nichtwässrigen polar-protischen Lösungsmitteln vorgenommen wird (vgl. Ausführungsbeispiel nach Methode 4, Fig. 5D). Ohne an eine Theorie gebunden zu sein, kann dies damit erklärt werden, dass in derartigen Fällen eine Substitution oberflächengebundener organischer Reste mit größeren Molmassen, etwa C₆- oder C₇-Reste, durch Reste erfolgt, die deutlich geringere Molmassen aufweisen, beispielsweise C₁- bis C₃-Reste. Diese stellen dann in der nachfolgenden Silanisierungsreaktion günstigere Abgangsgruppen dar, da dann beispielsweise im Rahmen der Silanisierungsreaktion mit Chlorsilanen leichtflüchtige C₁- bis C₃-Halogenide entstehen, die als Gase oder niedrigsiedende Flüssigkeiten leicht aus dem Reaktionsgefäß entweichen können, und dadurch eine Verschiebung des Reaktionsgleichgewichtes hin zu den gewünschten Produkten ermöglichen. Die Vorbehandlung mit Mischungen verschiedener polar-protischer Lösungsmittel fällt demzufolge naturgemäß ebenfalls in den Umfang der Erfindung sowie ihrer Äquivalente.

Anhand der vorstehend beschriebenen unterschiedlichen Effekte, die die erfindungsgemäße Vorbehandlung mit polar-protischen Lösungsmitteln aufweist (Entschützung von OH-Gruppen und Substitution von höhermolekularen organischen Reste durch niedermolekulare), insbesondere der daraus zwangsläufig resultierenden unterschiedlichen Reaktivität der freien OH-Gruppen und der "niedermolekular" substituierten OH-Gruppen in der nachfolgenden Silanisierungsreaktion, ist ohne weiteres ersichtlich, dass das erfindungsgemäße Verfahren die Einstellung sehr unterschiedlicher Silanisierungsgrade erlaubt. Belegt wird dies durch die bei den beispielhaft durchgeführten Thermogravimetrie-Messungen beobachteten großen Massenverluste von bis zu über 50% (vgl. Fig. 5B-E), die durch die Organischen Reste des Silylierungsmittel verursacht werden, und damit den Silanisierungsgrad belegen.

Nach der Vorbehandlung mit dem polar-protischen Lösungsmittel werden die Metalloxid-Nanopartikel durch Zugabe eines polar-aprotischen Fällungsmittels, etwa eines Ketons oder Ethers ausgefällt. Besonders geeignet sind dafür polar-aprotische Fällungsmittel, die mit dem verwendeten polar-protischen Lösungsmittel zumindest teilweise mischbar sind. Besonders geeignet ist Aceton. Es kann dafür aber auch ein polar-protisches Lösungsmittel, etwa ein Alkohol verwendet werden, der eine längere bzw. größere Alkylkette aufweist als dasjenige polar-protische Lösungsmittel, das für die Vorbehandlung verwendet wurde.

Sowohl bei den bisher beschriebenen Schritten Vorbehandlung und Ausfällung, als auch bei allen weiteren Schritten, insbesondere auch der Durchführung der Silanisierungsreaktion selbst, wird konsequent unter Ausschluss von Säuren und Basen gearbeitet, um die damit unvermeidlich einhergehende Agglomerisierung der Metalloxidpartikel (insbesondere Nanopartikel, aber auch bei größeren Partikeln stattfindend) zu vermeiden, die dazu führen würde, dass Oberflächenbereiche durch die Agglomerisierung vor und/oder während der Durchführung der Silanisierung gegenüber dieser "abgeschirmt" würden, so dass an ihnen keine Oberflächenmodifizierung, z. B. Silanisierung, stattfinden könnte.

Vorteilhafterweise müssen die erfindungsgemäß silanisierten (bzw. allgemein modifizierten) Nanopartikel für deren weitere Verwendung keiner weiteren Nachbehandlung wie beispielweise Dispergierung mehr unterzogen werden, sondern können direkt oder nach Aufbereitung, beispielsweise Aufkonzentrierung, weiterverarbeitet werden. Dies resultiert daraus, dass die Silanisierung erfindungsgemäß an der gesamten Oberfläche der nicht-agglomerierten Primärpartikeln erfolgt, einschließlich der durch die Vorbehandlung "entschützten" OH-Gruppen. Dieser technische Effekt der Erfindung, die "Entschützung" zuvor "geschützter", d.h. "blockierter" und damit zunächst nicht für die Oberflächenmodifizierung verfügbarer OH-Gruppen, ist skalenunabhängig, d.h. er tritt bei allen nano-, mikro- und makroskopischen Metalloxid-Oberflächen auf, die hydrolysierbare Gruppen aufweisen.

Die Gefahr, dass silanisierte Agglomerate während der nachfolgenden Verarbeitung absichtlich oder unabsichtlich wieder aufgebrochen werden, wodurch der erreichte Silanisierungsgrad der Partikeloberflächen durch Bildung neuer, nicht-silanisierter, Oberflächen wieder reduziert wird, ist durch das erfindungsgemäße Silanisierungsverfahren sicher unterbunden.

Die ausgefällten Nanopartikel werden anschließend von dem Gemisch aus Lösungsmittel und Fällungsmittel abgetrennt und in das für die Silanisierungsreaktion vorgesehene Lösungsmittel überführt. Bevorzugt handelt es sich dabei ebenfalls um polar-aprotische Lösungsmittel wie beispielsweise Tetrahydrofuran, 1,4-Dioxan u.a. Die Auswahl des Lösungsmittels für die Durchführung der Silanisierung erfolgt natürlich unter Berücksichtigung der jeweils konkret eingesetzten Silanisierungsreaktion und deren Erfordernisse, insbesondere hinsichtlich Edukten und ggf. Katalysatoren. Entsprechend umfangreich sind die Auswahlmöglichkeiten für das Lösungsmittel für die Silanisierung, ohne dabei den Umfang der Erfindung oder ihrer Äquivalente zu verlassen. Es fällt natürlich auch in den Umfang der Erfindung sowie ihrer Äquivalente, auf eine Abtrennung der ausgefällten Nanopartikel zu verzichten und die Silanisierungsreaktion direkt in der oben beschriebenen "Fällungs-Dispersion" durchzuführen, sofern dies die Erfordernisse der betreffenden Silanisierungsreaktion zulassen. Als "Fällungs-Dispersion" wird hierbei die Dispersion, oder auch Suspension, verstanden, die bereits durch Zugabe des Fällungsmittels und infolge des Ausfällungsprozesses erhalten wird. Diese Fällungs-Dispersion kann natürlich auch weiteren Dispergierschritten unterworfen werden. Der hier beschriebene Abtrennungs- und Überführungsschritt kann also entfallen, ohne den Umfang der Erfindung sowie ihrer Äquivalente zu verlassen. Auch einfaches Eindampfen der Fällungsdispersion bis zur Trockene, um ein pulverförmiges Präparat der vorbehandelten Metalloxid-Nanopartikel zu erhalten, ist möglich ohne den Umfang der Erfindung oder ihrer Äquivalente zu verlassen.

Im Rahmen des hier offenbarten Verfahrens zur chemischen Modifizierung von Metalloxidoberflächen können für die Durchführung der chemischen Modifizierung unterschiedlichste Modifizierungsreaktionen angewandt werden. Ausführlich beschrieben wird das Verfahren am Beispiel der Silanisierung, da diese ein besonders vorteilhaftes Ausführungsbeispiel darstellt. Es ist jedoch ohne weiteres für den Fachmann ersichtlich, dass a) auch andere Reaktionen für die Anknüpfung des Oberflächenmodifizierungsmittels verwendet werden können, b) das Oberflächenmodifizierungsmittel, je nachdem wie das oberflächenmodifizierte Metalloxid verwendet werden soll, unterschiedliche weitere reaktive Substituenten aufweisen kann, und dass c) das Oberflächenmodifizierungsmittel nicht zwingend einen (oder mehrere) weitere polymerisierbaren oder sonst wie reaktiven Substituenten zusätzlich zu demjenigen, mit dem die Anbindung an die Metalloxidoberfläche erfolgt, aufweisen muss. Wenn also hier sowie weiterhin von Silanisierung, Silanisierungsreaktion, Silanisierungsmittel und dergleichen mehr gesprochen wird, so ist dies jeweils beispielhaft als besonders vorteilhaftes Ausführungsbeispiel der Erfindung, jedoch in keiner Weise einschränkend zu verstehen. Isocyanatgruppen ("-NCO") beispielsweise können sowohl mit oberflächengebundenen OH-Gruppen reagieren und dadurch eine chemische Bindung eines Isocyanatgruppenhaltigen Oberflächen-Modifizierungsmittels an eine Metalloxidoberfläche bewirken, als auch Polymerisationsreaktionen mit Polyolen eingehen und dadurch die chemische Anbindung entsprechend modifizierter Metalloxid-Oberflächen an Polyurethan-Polymere bewirken. Auch in Polyamide lassen sich Isocyanatmodifizierte Metalloxid-Oberflächen chemisch "einpolymerisieren". Im Stand der Technik sind beispielsweise zahlreiche (Poly-)Isocyanate als Aktivatoren für die anionisch aktivierte Lactampolymerisation bekannt, die zu Polyamiden führt, zum Beispiel BRÜGGOLEN® C20P der Fa. Brüggemann Chemical. Ein Beispiel für die vorstehend genannte Variante c) ist schließlich der Fall, dass durch die Oberflächenmodifizierung keine Einbindung der Metalloxid-Nanopartikel in eine Polymermatrix erreicht werden soll, sondern lediglich beabsichtigt ist, die Oberfläche hydrophober zu machen. In diesem Falle kann als Oberflächen-Modifizierungsmittel beispielsweise ein Trialkyl-Siliciumchlorid eingesetzt werden, das via Hydrolyse der Si-Cl-Bindung durch eine OH-Gruppe der Metalloxid-Oberfläche an diese gebunden wird, wodurch sie infolge der Alkylreste am Si hydrophober wird. Anhand dieses Beispiels sei an dieser Stelle darüber hinaus erwähnt, dass es auch in den Rahmen der Erfindung sowie ihrer Äquivalente fällt, bei der Silanisierungsreaktion etwa entstehende Säuren oder Basen aktiv zu entfernen, bzw. zu neutralisieren, um eine zu starke pH-Wert-Änderung im Zuge der Silanisierungsreaktion zu vermeiden. Dazu kann die Silanisierungsreaktion ggf. in einer Pufferlösung durchgeführt werden.

Die eigentliche Silanisierung erfolgt somit durch die Umsetzung der Metalloxid-Nanopartikel in einem für die Umsetzung geeigneten Lösungsmittel unter Rühren, Schütteln oder sonstiger geeigneter Bewegung der Reaktionsmischung bei oder oberhalb von Raumtemperatur (typischerweise ca. 20 °C). Das Silanisierungsmittel kann entweder im Lösungsmittel vorgelegt und die vorbehandelten Nanopartikel zugegeben werden, oder umgekehrt. Auch die gleichzeitige Zugabe von Nanopartikel und Silanisierungsmittel ist möglich.

Dadurch, dass erfindungsgemäß unter Ausschluss von Säuren und Basen gearbeitet wird, stellt sich aufgrund des basischen oder sauren Charakters des Metalloxids selbst ein pH-Wert ein, der geringfügig vom isoelektrischen Punkt entfernt ist, bei dem die Metalloxid-Oberfläche elektrisch ungeladen bzw. elektrisch neutral ist. Diese minimale, quasi "ungestörte", Selbstaufladung der Partikeloberflächen aufgrund des sauren bzw. basischen Charakters in dem jeweiligen Lösungsmittel verhindert somit die Agglomeration, die beim isoelektrischen Punkt (ungeladene Partikeloberfläche) besonders effektiv eintreten würde. Infolge der ausbleibenden Agglomeration kann anschließend die Silanisierung weitestgehend ungestört an der gesamten Primärpartikel-Oberfläche erfolgen, sofern eine stärkere pH-Wert-Verschiebung möglichst vermieden wird, bzw. durch entsprechende geeignete Reaktionsführung erst eintritt, nachdem die Metalloxidoberfläche bereits vollständig mit dem Silanisierungsmittel - zumindest adsorptiv - belegt ist. Wie vorstehend bereits erläutert kann dazu in einer Pufferlösung gearbeitet werden.

Nachfolgend wird eine Auswahl besonders geeigneter Silanisierungsmittel beispielhaft aufgeführt, die jedoch in keiner Weise einschränkend zu verstehen ist.

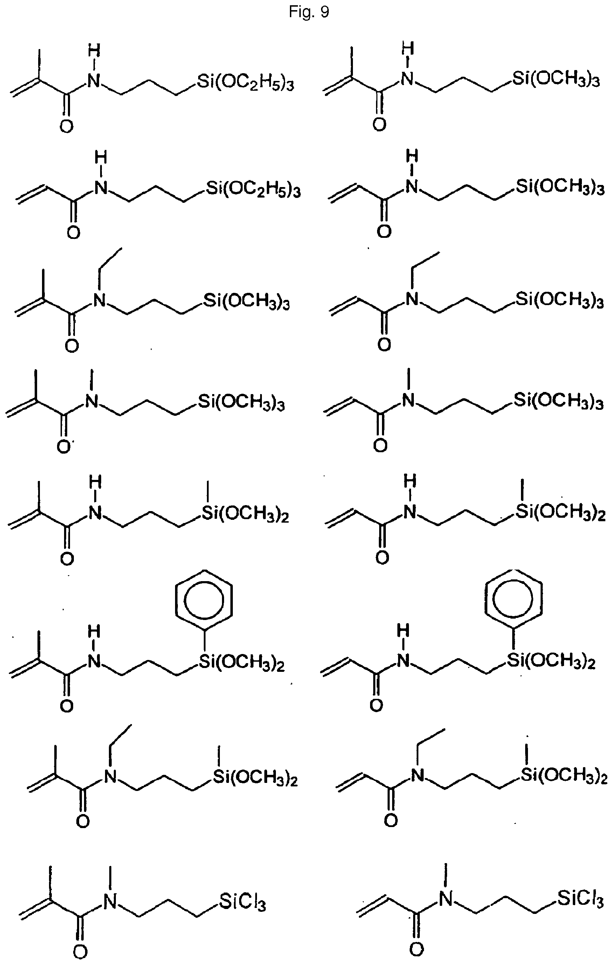

Bevorzugte Verbindungen (Silanisierungsmittel) der Formel (**I**) (s.u.), die erfindungsgemäß eingesetzt werden können, sind: (Meth)acrylamidoalkyltrialkoxysilane, insbesondere 3-(N-Methacryloylamino)-propyltrimethoxysilan, 3-(N-Acryloylamino)-propyltrimethoxysilan, 3-(N-Methacryloylamino)-propyltriethoxysilan, 3-(N-Methacryl-N-ethyl-amino)-propyltrimethoxysilan, 3-(N-Methacryl-N-ethylamino)-propyltrimethoxysilan, 3-(N-Acryl-N-ethylamino)-propyltrimethoxysilan, 3-(N-Methacryl-N-methyl-amino)-propyltrimethoxysilan, 3-(N-Acryl-N-methylamino)-propyltrimethoxysilan (vgl. Fig. 9).

Weitere bevorzugt erfindungsgemäß als Silanisierungsmittel einsetzbare Verbindungen sind (Meth)acrylamidoalkyl-alkyl- und -aryldialkoxysilane oder (Meth)acrylamidoalkyltrichlorsilane, insbesondere 3-(N-Methacryloylamino)-propylmethyldimethoxysilan, 3-(N-Acryloylamino)-propylmethyldimethoxysilan, 3-(N-Methacryloylamino)-propylphenyldimethoxysilan, 3-(N-Acryloylamino)-propylphenyldimethoxysilan, 3-(N-Methacryl-N-ethylamino)-propyl-methyldimethoxysilan, 3-(N-Acryl-N-ethylamino)-propyl-methyldimethoxysilan, 3-(N-Methacryl-N-methyl-amino)-propyltrichlorsilan und 3-(N-Acryl-N-methylamino)-propyltrichlorsilan (vgl. ebenfalls Fig. 9).

Dem Fachmann ist naheliegenderweise bekannt und es fällt daher in den Umfang der Erfindung, dass die vorstehend beschriebenen Silanisierungsmittel anstelle von radikalisch polymerisierbaren Substituenten auch andere reaktive Substituenten aufweisen können.

Die im wesentlichen pH-neutrale Vorbehandlung und Bereitstellung der Metalloxid-Nanopartikel vermeidet somit die im Stand der Technik auftretende Agglomeratbildung aufgrund der Säure-/Basenbehandlung, so dass im nachfolgenden eigentlichen Silanisierungsschritt die Oberfläche sehr viel gleichmäßiger modifiziert wird.

Die erfindungsgemäß oberflächenmodifizierten Metalloxid-Nanopartikel sind sehr gut geeignet zur Verwendung als Füllstoffe in Polymeren. Bei Verwendung einer Verbindung der Formel (**I**) als Silanisierungsmittel eignen sie sich ganz besonders für die Verwendung als Füllstoffe in Dentalwerkstoffen/Dentalmaterialien, bzw. Dentalkompositen. Dazu werden die erfindungsgemäß silanisierten Metalloxid-Nanopartikel - wie beispielhaft weiter unten beschrieben - mit einer radikalisch polymerisierbaren Monomerverbindung oder einer Mischung von mindestens zwei radikalisch polymerisierbaren Monomerverbindungen und ggf. einem oder mehreren Initiatoren sowie ggf. weiteren Monomerbestandteilen für die Durchführung der radikalischen Polymerisation gemischt und polymerisiert. Die weiteren Monomerbestandteile dienen dabei beispielsweise der Einstellung der Prozessierbarkeit der Monomermischung, z. B. der Fließfähigkeit u.a., und/oder der Einstellung der Materialeigenschaften (z.B. Brechzahl, Zähigkeit, Schlagfestigkeit, Härte) des fertig polymerisierten Kompositmaterials.

Bevorzugte Monomerverbindungen sind hydrolysestabile Verbindungen, wie hydrolysestabile Mono(meth)acrylate, z.B. Mesitylmethacrylat, N-mono- oder N,N-disubstitiuierten Acrylamide, wie z.B. N-Ethylacrylamid, N,N-Dimethacrylamid, N-(2-Hydroxyethyl)acrylamid und N-Methyl-N-(2-hydroxyethyl)acrylamid, N-monosubstituierte Methacrylamide, wie z.B. N-Ethylmethacrylamid und N-(2-Hydroxyethyl)methacrylamid, sowie N-Vinylpyrrolidon. Als hydrolysestabil werden solche Monomere bezeichnet, die in Wasser oder in Mischungen von Wasser und wassermischbaren Lösungsmitteln bei einer Konzentration von ca. 20 Gew.-% und einem pH-Wert von ca. 2,0 bei 37°C für mindestens 6 Wochen stabil sind, d.h. zu weniger als 5 % hydrolysieren.

Weiterhin sind auch quervernetzbare Monomerverbindungen bevorzugt, d.h. Monomere mit zwei oder mehr, vorzugsweise 2 bis 4 radikalisch polymerisierbaren Gruppen. Bevorzugte quervernetzbare Monomerverbindungen sind vernetzende Pyrrolidone, wie z.B. 1,6-Bis(3-vinyl-2-pyrrolidonyl)-hexan, oder kommerziell zugängliche Bisacrylamide, wie Methylen- oder Ethylenbisacrylamid, Bis(meth)-acrylamide, wie z.B. N,N'-Diethyl-1,3-bis(acrylamido)-propan, 1,3-Bis(methacrylamido)-propan, 1,4-Bis(methacrylamido)-butan, 1,4-Bis(acrylamido)-butan und 1,4-Bis(acrylamido)-piperazin, die durch Umsetzung aus den entsprechenden Diaminen mit (Meth)acrylsäurechorid synthetisiert werden können. Alle vorstehend genannten Monomerarten können allein oder in Kombination miteinander eingesetzt werden.

Zusammenfassend lässt sich die Erfindung daher wie folgt beschreiben, wobei beispielsweise Bereichsangaben stets alle - nicht genannten - Zwischenwerte und alle denkbaren Teilintervalle umfassen.

Der Gegenstand der Erfindung umfasst folgendes Verfahren zur Silanisierung von Metalloxid-Nanopartikeln:
a) Vorbehandlung der Metalloxid-Nanopartikel mit einem polar-protischen Lösungsmittel, umfassend die Dispergierung der Metalloxid-Nanopartikel in dem polar-protischen Lösungsmittel, ausgewählt aus der Liste umfassend Wasser, Methanol, Ethanol, Propanol, Butanol, so dass eine Dispersion entsteht;
b) Fällung der Metalloxid-Nanopartikel aus der in Schritt a) erhaltenen Dispersion entweder i) durch Zugabe eines polar-aprotischen Fällungsmittels, ausgewählt aus der Liste umfassend Ketone, Aceton, Methylethylketon, Ether, Tetrahydrofuran, 1,4-Dioxan, Trioxan, wobei das polar-aprotische Fällungsmittel mit dem polar-protischen Lösungsmittel aus Schritt a) zumindest teilweise mischbar ist, oder ii) durch Zugabe eines polar-protischen Fällungsmittels, das einen größeren Alkylrest aufweist als das in Schritt a) verwendete polar-protische Lösungsmittel, und das mit dem polar-protischen Lösungsmittel aus Schritt a) zumindest teilweise mischbar ist;
c) Abtrennung der in Schritt b) ausgefällten Metalloxid-Nanopartikel von der flüssigen Phase und Überführung in ein bereitgestelltes Lösungsmittel für die Durchführung der Silanisierung im nachfolgenden Schritt d), wobei dieses Lösungsmittel für die Silanisierung ausgewählt ist aus der Liste umfassend Tetrahydrofuran und 1,4-Dioxan, oder es sich um ein anderes polar-aprotisches Lösungsmittel handelt;
d) Umsetzung der gemäß Schritt c) abgetrennten Metalloxid-Nanopartikel in dem bereitgestellten Lösungsmittel für die Silanisierung mit einem Silanisierungsmittel, wobei die Zugabe des Silanisierungsmittels vor und/oder während und/oder nach der Überführung der Metalloxid-Nanopartikel in das bereitgestellte Lösungsmittel gemäß Schritt c) erfolgt.

Die Schritte a) bis d) erfolgen unter Ausschluss von Säuren und Basen bei denjenigen pH-Werten, die sich durch den Kontakt der vorbehandelten Metalloxid-Nanopartikel in der Reaktionslösung bzw. Dispersion einstellen, d.h. bei pH-Werten zwischen pH = 2 bis pH = 10, bevorzugt bei pH-Werten zwischen pH = 4 bis pH = 9, und ganz besonders bevorzugt bei im wesentlichen neutralem pH-Wert im Bereich von etwa pH = 6 bis etwa pH = 8.

Der Gegenstand der Erfindung umfasst weiterhin eine Variante des Verfahrens wie vorstehend beschrieben, die sich dadurch auszeichnet, dass die Abtrennung gemäß Schritt c) mindestens eines der Trennungsverfahren umfasst, ausgewählt aus der Liste, umfassend Filtration, Zentrifugation, Sedimentation, Dialyse, Destillation, Trocknung, wobei diese Verfahren einzeln, mehrfach hintereinander, in beliebiger Kombination miteinander sowie mit oder ohne vor-, zwischen- oder nachgeschalteten Waschprozessen vorgenommen werden.

Der Gegenstand der Erfindung umfasst weiterhin eine Variante des Verfahrens wie vorstehend beschrieben, die sich dadurch auszeichnet, dass das Fällungsmittel gemäß Schritt b) ii) ausgewählt ist aus der Liste umfassend Propanol, iso-Propanol, Butanol, iso-Butanol, tert.-Butanol, Pentanol, iso-Pentanol, tert.-Pentanol, Hexanole, Heptanole, Glykole, Diglykol, Triglykol.

Der Gegenstand der Erfindung umfasst weiterhin eine Variante des Verfahrens wie vorstehend beschrieben, die sich dadurch auszeichnet, dass Schritt a) und/oder Schritt d) unter Einstrahlung von Mikrowellenstrahlung und/oder unter Beschallung mit Ultraschall durchgeführt wird.

Der Gegenstand der Erfindung umfasst weiterhin eine Variante des Verfahrens wie vorstehend beschrieben, die sich dadurch auszeichnet, dass Schritt d) in einer Pufferlösung durchgeführt wird, so dass durch die Silanisierungsreaktion entstehende Säuren oder Basen neutralisiert werden.

Der Gegenstand der Erfindung umfasst weiterhin eine Variante des Verfahrens wie vorstehend beschrieben, die sich dadurch auszeichnet, dass sich an Schritt d) noch mindestens ein weiterer Schritt e) zur Aufarbeitung der gemäß Schritt d) erhaltenen Dispersion von silanisierten Metalloxid-Nanopartikeln anschließt, der mindestens eines der Aufarbeitungsverfahren umfasst, ausgewählt aus der Liste, umfassend Filtration, Zentrifugation, Sedimentation, Dialyse, Destillation, Trocknung.

Der Gegenstand der Erfindung umfasst weiterhin eine Variante des Verfahrens wie vorstehend beschrieben, die sich dadurch auszeichnet, dass es sich bei den Metalloxid-Nanopartikeln um Metalloxid-Nanopartikel handelt, die vor oder nach der Silanisierung eine mittlere Partikelgröße zwischen 500 nm und 0,5 nm, bevorzugt zwischen 200 nm und 1 nm, besonders bevorzugt zwischen 1,5 nm und 20 nm aufweisen.

Der Gegenstand der Erfindung umfasst weiterhin eine Variante des Verfahrens wie vorstehend beschrieben, die sich dadurch auszeichnet, dass es sich bei den Metalloxid-Nanopartikeln um ZrO₂-Nanopartikel oder um TiO₂-Nanopartikel handelt.

Der Gegenstand der Erfindung umfasst weiterhin eine Variante des Verfahrens wie vorstehend beschrieben, die sich dadurch auszeichnet, dass es sich bei den Metalloxid-Nanopartikeln um Metalloxid-Nanopartikel handelt, enthaltend mindestens ein Metall oder Halbmetall, ausgewählt aus der Liste umfassend die Metalle bzw. Halbmetalle In, Zr, Ti, Ge, Sn, Pb, Sb, Si. Bezüglich letzterem (Si), dessen Oxid ja bereits - wie weiter oben beschrieben - in größerem Umfange Hydroxylgruppen aufweist als andere Oxide, bewirkt das erfindungsgemäße Verfahren dennoch eine weitere Verbesserung der Silanisierbarkeit/Funktionalisierbarkeit, so daß Si vom Umfang der Erfindung sowie ihrer Äquivalente mit umfasst ist.

Der Gegenstand der Erfindung umfasst weiterhin eine Variante des Verfahrens wie vorstehend beschrieben, die sich dadurch auszeichnet, dass die Metalloxid-Nanopartikel eine Dotierung aufweisen, die mindestens ein Element umfasst, ausgewählt aus der Liste umfassend die chemischen Elemente Cu, Ag, Au, Co, Fe, Co, Ni, Ru, Rh, Pd, Os, Ir, Pt, N, S, P, Sc, Y, Lanthanoide (Seltenerdmetalle), Sr, Ba, Rb, Cs, Li.

Der Gegenstand der Erfindung umfasst weiterhin eine Variante des Verfahrens wie vorstehend beschrieben, die sich dadurch auszeichnet, dass es sich bei dem Silanisierungsmittel um ein Silanisierungsmittel handelt, umfassend mindestens eine Verbindung der Formel (**I**),

[(PG)-R₁-Z]ₙ SP [R₂-(AG)]_{m,} (**I**)

wobei
AG SiR3R4X ist,
R₁ eine C₁-C₃-Alkylengruppe oder eine substituierte oder unsubstituierte Cyclopropylengruppe ist oder entfällt,
R₂ eine C₁-C₁₀-Alkylengruppe ist oder entfällt,
R₃ eine C₁-C₈-Alkylgruppe, Chlor oder OR₅ ist,
R₄ eine C₁-C₈-Alkylgruppe, Phenyl, Chlor oder OR₅ ist,
R₅ eine C₁-C₆-Alkylgruppe ist,
X OR₅ oder Chlor ist,
Z CO-NR₆ ist, wobei R₆ H oder C₁-C₆-Alkyl ist,
PG eine radikalisch polymerisierbare Gruppe der Formel (**II**), 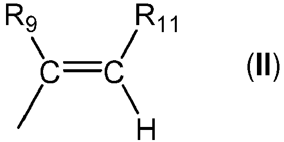
ist, in der R₉ H, C₁-C₃-Alkyl, C₁-C₃-Hydroxyalkyl oder COOR₁₀ ist, wobei R₁₀ H, C₁-C₁₀-Alkyl, 1,6-Dimethylphenyl oder Mesityl ist,
R₁₁ H oder Phenyl ist,
m 1 oder 2 ist,
n 1, 2, 3 oder 4 ist,
SP entfällt oder ein (n+m)-wertiger linearer oder verzweigter aliphatischer C₁-C₃₀-Rest ist, bei dem die Kohlenstoffkette durch O, S, CO-NH, O-CO-NH oder NH-CO-NH unterbrochen sein kann, oder ein (n+m)-wertiger aromatischer C₆-C₁₈-Rest ist, oder ein (n+m)-wertiger cycloaliphatischer C₃-C₁₈-Rest ist, oder ein (n+m)-wertiger heterocyclischer C₃-C₁₈-Rest ist, wobei die Reste unsubstituiert oder durch C₁-C₅-Alkyl, Cl, Br und/oder OH substituiert sein können.

Die Angabe, dass ein Rest durch Fremdatome oder Gruppen, wie Sauerstoff oder Schwefel, unterbrochen sein kann, ist so zu verstehen, dass eines oder mehrere der Fremdatome oder eine oder mehrere der Gruppen in eine Kohlenstoffkette integriert sind. Daraus folgt, dass die Fremdatome oder Gruppen nicht endständig sein können, d.h. eine Anbindung an Nachbargruppen immer über ein Kohlenstoffatom erfolgt, und dass die Zahl der Fremdatome und Gruppen zwangsläufig kleiner als die Zahl der Kohlenstoffatome sein muß. Formel (**I**) erstreckt sich nur auf solche Verbindungen, die mit der chemischen Valenzlehre vereinbar sind. Bevorzugte polymerisationsfähige Gruppen PG sind Vinylgruppen der Formel H₂C=C(-R₉)-, Acrylsäuregruppen der Formel H₂C=C(-COOR₁₀)- sowie Allyl-, Styryl- und/oder Vinylcyclopropyl-Gruppen. Bevorzugte Definitionen der obigen Variablen, die unabhängig voneinander gewählt werden können, sind:
R₁ = eine Methylengruppe, Cyclopropylengruppe oder entfällt,
R₂ = eine C₁-C₃-Alkylengruppe oder entfällt,
R₃ = eine C₁-C₃-Alkylgruppe, Chlor, insbesondere ORs,
R₄ = eine C₁-C₃-Alkylgruppe, Phenyl, Chlor, insbesondere ORs,
R5 = eine C₁-C₂-Alkylgruppe,
R₆ = H, eine C₁-C₃-Alkylgruppe, insbesondere Methyl,
X = Chlor, insbesondere ORs,
PG = eine Vinylgruppe H₂C=C(R₉)-, in der R₉ = H oder R₉ = CH₃ ist,
m = 1 oder 2,
n = 1 oder 2,
SP = entfällt oder ein (n+m)-wertiger linearer oder verzweigter aliphatischer C₁-C₆-Rest, ein (n+m)-wertiger aromatischer C₆-C₁₀-Rest, ein (n+m)-wertiger cycloaliphatischer C₃-C₁₀-Rest oder ein (n+m)-wertiger heterocyclischer C₃-C₁₀-Rest, wobei die Reste substituiert oder unsubstituiert sein können. Ganz besonders bevorzugt sind Verbindungen, bei denen mindestens zwei und insbesondere alle Variablen eine der bevorzugten Bedeutungen haben.

Der Gegenstand der Erfindung umfasst weiterhin eine Variante des weiter oben beschriebenen Verfahrens, die sich dadurch auszeichnet, dass es sich bei dem Silanisierungsmittel um 3-(Trimethoxysilyl)propylmethacrylat handelt.

Der Gegenstand der Erfindung umfasst weiterhin silanisierte Metalloxid-Nanopartikel, hergestellt nach einem Verfahren wie vorstehend beschrieben, die sich dadurch auszeichnen, dass sie bei thermogravimetrischen Messungen bis zu einer Temperatur von 800 °C in synthetischer Luft einen Gesamt-Massenverlust von 10 bis 60 Prozent, bevorzugt von 10 bis 50 Prozent und besonders bevorzugt von 15 bis 40 Prozent, aufweisen.

Der Gegenstand der Erfindung umfasst weiterhin die Verwendung von erfindungsgemäß silanisierten Metalloxid-Nanopartikeln, bzw. von erfindungsgemäßen Metalloxid-Nanopartikeln, als Füllstoff in Polymeren.

Der Gegenstand der Erfindung umfasst weiterhin die Verwendung von erfindungsgemäß silanisierten Metalloxid-Nanopartikeln, bzw. von erfindungsgemäßen Metalloxid-Nanopartikeln, als Füllstoff in Dentalkompositen.

Weiterhin lässt sich die Erfindung in der Ausführungsvariante für größere Metalloxidpartikel wie folgt beschreiben:
Der Gegenstand der Erfindung umfasst folgendes Verfahren zur Silanisierung von Metalloxidpartikeln:
a) Vorbehandlung der Metalloxidpartikel mit einem polar-protischen Lösungsmittel, umfassend die Dispergierung/Suspendierung der Metalloxidpartikel in dem polar-protischen Lösungsmittel, ausgewählt aus der Liste umfassend Wasser, Methanol, Ethanol, Propanol, Butanol, so dass eine Dispersion/Suspension entsteht;
b) Unterstützung der Sedimentation der Metalloxidpartikel aus der in Schritt a) erhaltenen Dispersion/Suspension entweder i) durch Zugabe eines polar-aprotischen Sedimentierungshilfsmittels, ausgewählt aus der Liste umfassend Ketone, Aceton, Methylethylketon, Ether, Tetrahydrofuran, 1,4-Dioxan, Trioxan, wobei das polar-aprotische Sedimentierungshilfsmittel mit dem polar-protischen Lösungsmittel aus Schritt a) zumindest teilweise mischbar ist, oder ii) durch Zugabe eines polar-protischen Sedimentierungshilfsmittels, das einen größeren Alkylrest aufweist als das in Schritt a) verwendete polar-protische Lösungsmittel, und das mit dem polar-protischen Lösungsmittel aus Schritt a) zumindest teilweise mischbar ist;
c) Abtrennung der in Schritt b) sedimentierten Metalloxidpartikel von der flüssigen Phase und Überführung in ein bereitgestelltes Lösungsmittel für die Durchführung der Silanisierung im nachfolgenden Schritt d), wobei dieses Lösungsmittel für die Silanisierung ausgewählt ist aus der Liste umfassend Tetrahydrofuran und 1,4-Dioxan, oder es sich um ein anderes polar-aprotisches Lösungsmittel handelt;
d) Umsetzung der gemäß Schritt c) abgetrennten Metalloxidpartikel in dem bereitgestellten Lösungsmittel für die Silanisierung mit einem Silanisierungsmittel, wobei die Zugabe des Silanisierungsmittels vor und/oder während und/oder nach der Überführung der Metalloxidpartikel in das bereitgestellte Lösungsmittel gemäß Schritt c) erfolgt.

Der in Schritt b) im Falle von Metalloxid-Nanopartikeln erfolgenden Fällung durch Zugabe eines Fällungsmittels entspricht im Falle von größeren Metalloxidpartikeln die Unterstützung der Sedimentation durch Zugabe eines Sedimentierungshilfsmittels.

Die Schritte a) bis d) erfolgen unter Ausschluss von Säuren und Basen bei denjenigen pH-Werten, die sich durch den Kontakt der vorbehandelten Metalloxidpartikel in der Reaktionslösung bzw. Dispersion einstellen, d.h. bei pH-Werten zwischen pH = 2 bis pH = 10, bevorzugt bei pH-Werten zwischen pH = 4 bis pH = 9, und ganz besonders bevorzugt bei im wesentlichen neutralem pH-Wert im Bereich von etwa pH = 6 bis etwa pH = 8.

Der Gegenstand der Erfindung umfasst weiterhin eine Variante des Verfahrens wie vorstehend beschrieben, die sich dadurch auszeichnet, dass die Abtrennung gemäß Schritt c) mindestens eines der Trennungsverfahren umfasst, ausgewählt aus der Liste, umfassend Filtration, Zentrifugation, Sedimentation, Dialyse, Destillation, Trocknung, wobei diese Verfahren einzeln, mehrfach hintereinander, in beliebiger Kombination miteinander sowie mit oder ohne vor-, zwischen- oder nachgeschalteten Waschprozessen vorgenommen werden.

Der Gegenstand der Erfindung umfasst weiterhin eine Variante des Verfahrens wie vorstehend beschrieben, die sich dadurch auszeichnet, dass das Sedimentierungshilfsmittel gemäß Schritt b) ii) ausgewählt ist aus der Liste umfassend Propanol, iso-Propanol, Butanol, iso-Butanol, tert.-Butanol, Pentanol, iso-Pentanol, tert.-Pentanol, Hexanole, Heptanole, Glykole, Diglykol, Triglykol.

Der Gegenstand der Erfindung umfasst weiterhin eine Variante des Verfahrens wie vorstehend beschrieben, die sich dadurch auszeichnet, dass Schritt a) und/oder Schritt d) unter Einstrahlung von Mikrowellenstrahlung und/oder unter Beschallung mit Ultraschall durchgeführt wird.

Der Gegenstand der Erfindung umfasst weiterhin eine Variante des Verfahrens wie vorstehend beschrieben, die sich dadurch auszeichnet, dass Schritt d) in einer Pufferlösung durchgeführt wird, so dass durch die Silanisierungsreaktion entstehende Säuren oder Basen neutralisiert werden.

Der Gegenstand der Erfindung umfasst weiterhin eine Variante des Verfahrens wie vorstehend beschrieben, die sich dadurch auszeichnet, dass sich an Schritt d) noch mindestens ein weiterer Schritt e) zur Aufarbeitung der gemäß Schritt d) erhaltenen Dispersion/Suspension von silanisierten Metalloxidpartikeln anschließt, der mindestens eines der Aufarbeitungsverfahren umfasst, ausgewählt aus der Liste, umfassend Filtration, Zentrifugation, Sedimentation, Dialyse, Destillation, Trocknung.

Der Gegenstand der Erfindung umfasst weiterhin eine Variante des Verfahrens wie vorstehend beschrieben, die sich dadurch auszeichnet, dass es sich bei den Metalloxidpartikeln um Metalloxidpartikel handelt, die vor oder nach der Silanisierung eine mittlere Partikelgröße zwischen 500 µm und 0,5 µm, bevorzugt zwischen 200 µm und 1 µm, besonders bevorzugt zwischen 1,5 µm und 20 µm aufweisen.

Der Gegenstand der Erfindung umfasst weiterhin eine Variante des Verfahrens wie vorstehend beschrieben, die sich dadurch auszeichnet, dass es sich bei den Metalloxidpartikeln um ZrO₂-Partikel oder um TiO₂-Partikel handelt.

Der Gegenstand der Erfindung umfasst weiterhin eine Variante des Verfahrens wie vorstehend beschrieben, die sich dadurch auszeichnet, dass es sich bei den Metalloxidpartikeln um Metalloxidpartikel handelt, enthaltend mindestens ein Metall oder Halbmetall, ausgewählt aus der Liste umfassend die Metalle bzw. Halbmetalle In, Zr, Ti, Ge, Sn, Pb, Sb, Si. Bezüglich letzterem (Si), dessen Oxid ja bereits - wie weiter oben beschrieben - in größerem Umfange Hydroxylgruppen aufweist als andere Oxide, bewirkt das erfindungsgemäße Verfahren dennoch eine weitere Verbesserung der Silanisierbarkeit/Funktionalisierbarkeit, so daß Si vom Umfang der Erfindung sowie ihrer Äquivalente mit umfasst ist.

Der Gegenstand der Erfindung umfasst weiterhin eine Variante des Verfahrens wie vorstehend beschrieben, die sich dadurch auszeichnet, dass die Metalloxidpartikel eine Dotierung aufweisen, die mindestens ein Element umfasst, ausgewählt aus der Liste umfassend die chemischen Elemente Cu, Ag, Au, Co, Fe, Co, Ni, Ru, Rh, Pd, Os, Ir, Pt, N, S, P, Sc, Y, Lanthanoide (Seltenerdmetalle), Sr, Ba, Rb, Cs, Li.

Der Gegenstand der Erfindung umfasst weiterhin eine Variante des Verfahrens wie vorstehend beschrieben, die sich dadurch auszeichnet, dass es sich bei dem Silanisierungsmittel um ein Silanisierungsmittel handelt, umfassend mindestens eine Verbindung der Formel (**I**),

[(PG)-R₁-Z]ₙ SP [R₂-(AG)]_{m,} (**I**)

wobei
AG SiR3R4X ist,
R₁ eine C₁-C₃-Alkylengruppe oder eine substituierte oder unsubstituierte Cyclopropylengruppe ist oder entfällt,
R₂ eine C₁-C₁₀-Alkylengruppe ist oder entfällt,
R₃ eine C₁-C₈-Alkylgruppe, Chlor oder OR₅ ist,
R₄ eine C₁-C₈-Alkylgruppe, Phenyl, Chlor oder OR₅ ist,
R₅ eine C₁-C₆-Alkylgruppe ist,
X OR₅ oder Chlor ist,
Z CO-NR₆ ist, wobei R₆ H oder C₁-C₆-Alkyl ist,
PG eine radikalisch polymerisierbare Gruppe der Formel (**II**), 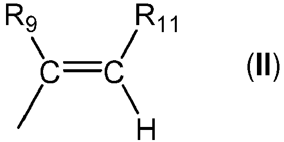
ist, in der R₉ H, C₁-C₃-Alkyl, C₁-C₃-Hydroxyalkyl oder COOR₁₀ ist, wobei R₁₀ H, C₁-C₁₀-Alkyl, 1,6-Dimethylphenyl oder Mesityl ist,
R₁₁ H oder Phenyl ist,
m 1 oder 2 ist,
n 1, 2, 3 oder 4 ist,
SP entfällt oder ein (n+m)-wertiger linearer oder verzweigter aliphatischer C₁-C₃₀-Rest ist, bei dem die Kohlenstoffkette durch O, S, CO-NH, O-CO-NH oder NH-CO-NH unterbrochen sein kann, oder ein (n+m)-wertiger aromatischer C₆-C₁₈-Rest ist, oder ein (n+m)-wertiger cycloaliphatischer C₃-C₁₈-Rest ist, oder ein (n+m)-wertiger heterocyclischer C₃-C₁₈-Rest ist, wobei die Reste unsubstituiert oder durch C₁-C₅-Alkyl, Cl, Br und/oder OH substituiert sein können.

Die Angabe, dass ein Rest durch Fremdatome oder Gruppen, wie Sauerstoff oder Schwefel, unterbrochen sein kann, ist so zu verstehen, dass eines oder mehrere der Fremdatome oder eine oder mehrere der Gruppen in eine Kohlenstoffkette integriert sind. Daraus folgt, dass die Fremdatome oder Gruppen nicht endständig sein können, d.h. eine Anbindung an Nachbargruppen immer über ein Kohlenstoffatom erfolgt, und dass die Zahl der Fremdatome und Gruppen zwangsläufig kleiner als die Zahl der Kohlenstoffatome sein muß. Formel (**I**) erstreckt sich nur auf solche Verbindungen, die mit der chemischen Valenzlehre vereinbar sind. Bevorzugte polymerisationsfähige Gruppen PG sind Vinylgruppen der Formel H₂C=C(-R₉)-, Acrylsäuregruppen der Formel H₂C=C(-COOR₁₀)- sowie Allyl-, Styryl- und/oder Vinylcyclopropyl-Gruppen. Bevorzugte Definitionen der obigen Variablen, die unabhängig voneinander gewählt werden können, sind:
R₁ = eine Methylengruppe, Cyclopropylengruppe oder entfällt,
R₂ = eine C₁-C₃-Alkylengruppe oder entfällt,
R₃ = eine C₁-C₃-Alkylgruppe, Chlor, insbesondere ORs,
R₄ = eine C₁-C₃-Alkylgruppe, Phenyl, Chlor, insbesondere ORs,
R5 = eine C₁-C₂-Alkylgruppe,
R₆ = H, eine C₁-C₃-Alkylgruppe, insbesondere Methyl,
X = Chlor, insbesondere ORs,
PG = eine Vinylgruppe H₂C=C(R₉)-, in der R₉ = H oder R₉ = CH₃ ist,
m = 1 oder 2,
n = 1 oder 2,
SP = entfällt oder ein (n+m)-wertiger linearer oder verzweigter aliphatischer C₁-C₆-Rest, ein (n+m)-wertiger aromatischer C₆-C₁₀-Rest, ein (n+m)-wertiger cycloaliphatischer C₃-C₁₀-Rest oder ein (n+m)-wertiger heterocyclischer C₃-C₁₀-Rest, wobei die Reste substituiert oder unsubstituiert sein können. Ganz besonders bevorzugt sind Verbindungen, bei denen mindestens zwei und insbesondere alle Variablen eine der bevorzugten Bedeutungen haben.

Der Gegenstand der Erfindung umfasst weiterhin eine Variante des weiter oben beschriebenen Verfahrens, die sich dadurch auszeichnet, dass es sich bei dem Silanisierungsmittel um 3-(Trimethoxysilyl)propylmethacrylat handelt.

Der Gegenstand der Erfindung umfasst weiterhin silanisierte Metalloxidpartikel, hergestellt nach einem Verfahren wie vorstehend beschrieben, die sich dadurch auszeichnen, dass sie bei thermogravimetrischen Messungen bis zu einer Temperatur von 800 °C in synthetischer Luft einen Gesamt-Massenverlust von 5 bis 30 Prozent, bevorzugt von 10 bis 25 Prozent und besonders bevorzugt von 15 bis 20 Prozent, aufweisen.

Der Gegenstand der Erfindung umfasst weiterhin die Verwendung von erfindungsgemäß silanisierten Metalloxidpartikeln, bzw. von erfindungsgemäßen Metalloxidpartikeln, als Füllstoff in Polymeren.

Der Gegenstand der Erfindung umfasst weiterhin die Verwendung von erfindungsgemäß silanisierten Metalloxidpartikeln, bzw. von erfindungsgemäßen Metalloxidpartikeln, als Füllstoff in Dentalkompositen.

Weiterhin lässt sich die Erfindung in der Ausführungsvariante für Metalloxidoberflächen wie folgt beschreiben:
Der Gegenstand der Erfindung umfasst folgendes Verfahren zur Silanisierung von Metalloxidoberflächen:
a) Vorbehandlung der Metalloxidoberfläche mit einem polar-protischen Lösungsmittel, umfassend die Benetzung der Metalloxidoberfläche mit dem polar-protischen Lösungsmittel, ausgewählt aus der Liste umfassend Wasser, Methanol, Ethanol, Propanol, Butanol, so dass die Metalloxidoberfläche benetzt ist;
b) Abspülen der in Schritt a) erhaltenen benetzten Metalloxidoberfläche entweder i) durch Benetzen mit einer polar-aprotischen Spülflüssigkeit, ausgewählt aus der Liste umfassend Ketone, Aceton, Methylethylketon, Ether, Tetrahydrofuran, 1,4-Dioxan, Trioxan, wobei die polar-aprotische Spülflüssigkeit mit dem polar-protischen Lösungsmittel aus Schritt a) zumindest teilweise mischbar ist, oder ii) durch Benetzen mit einer polar-protischen Spülflüssigkeit, die einen größeren Alkylrest aufweist als das in Schritt a) verwendete polar-protische Lösungsmittel, und die mit dem polar-protischen Lösungsmittel aus Schritt a) zumindest teilweise mischbar ist;
c) Überführung der gemäß Schritt b) abgespülten Metalloxidoberfläche in ein bereitgestelltes Lösungsmittel, bzw. Benetzung damit, für die Durchführung der Silanisierung im nachfolgenden Schritt d), wobei dieses Lösungsmittel für die Silanisierung ausgewählt ist aus der Liste umfassend Tetrahydrofuran und 1,4-Dioxan, oder es sich um ein anderes polar-aprotisches Lösungsmittel handelt;
d) Umsetzung der gemäß Schritt c) überführten Metalloxidoberfläche in dem bereitgestellten Lösungsmittel für die Silanisierung mit einem Silanisierungsmittel, wobei die Zugabe des Silanisierungsmittels vor und/oder während und/oder nach der Überführung der Metalloxidoberfläche in das bereitgestellte Lösungsmittel gemäß Schritt c) erfolgt.

Der in Schritt b) im Falle von Metalloxid-Nanopartikeln erfolgenden Fällung durch Zugabe eines Fällungsmittels entspricht im Falle von Metalloxidoberflächen das Abspülen der mit dem Lösungsmittel aus Schritt a) benetzten Metalloxidoberfläche durch Benetzung mit der Spülflüssigkeit. Sämtliche dem Fachmann bekannten Benetzungsverfahren, wie beispielsweise Besprühen, Eintauchen, Bestreichen usw., können grundsätzlich in allen entsprechenden Verfahrensschritten, die eine Benetzung vorsehen, verwendet werden, ohne den Umfang der Erfindung oder ihrer Äquivalente zu verlassen.

Der in Schritt c) im Falle von Metalloxid-Nanopartikeln erfolgenden Abtrennung der Metalloxid-Nanopartikel aus der Dispersion entspricht im Falle von Metalloxidoberflächen das Überführen der Metalloxidoberfläche, d.h. des betreffenden Gegenstandes, der die Metalloxidoberfläche aufweist, in das Lösungsmittel für die Silanisierung, bzw. die Benetzung der Metalloxidoberfläche damit. Diese Überführung bzw. Benetzung muß nicht zwingend die gesamte Metalloxidoberfläche betreffen, sondern kann sich auf Teilbereiche beschränken, die silanisiert werden sollen.

Die Schritte a) bis d) erfolgen unter Ausschluss von Säuren und Basen bei denjenigen pH-Werten, die sich durch den Kontakt der vorbehandelten Metalloxidoberfläche in der Reaktionslösung bzw. Dispersion einstellen, d.h. bei pH-Werten zwischen pH = 2 bis pH = 10, bevorzugt bei pH-Werten zwischen pH = 4 bis pH = 9, und ganz besonders bevorzugt bei im wesentlichen neutralem pH-Wert im Bereich von etwa pH = 6 bis etwa pH = 8.

Der Gegenstand der Erfindung umfasst weiterhin eine Variante des Verfahrens wie vorstehend beschrieben, die sich dadurch auszeichnet, dass die Überführung gemäß Schritt c) Teilschritte umfasst, wie etwa Trocknen durch Wärmeeinwirkung oder Anblasen mit Luft oder anderen Gasen bzw. Gasmischungen, mechanisches Abwischen, Anlegen von Vakuum an die Metalloxidoberfläche u.a.m.

Der Gegenstand der Erfindung umfasst weiterhin eine Variante des Verfahrens wie vorstehend beschrieben, die sich dadurch auszeichnet, dass die Spülflüssigkeit gemäß Schritt b) ii) ausgewählt ist aus der Liste umfassend Propanol, iso-Propanol, Butanol, iso-Butanol, tert.-Butanol, Pentanol, iso-Pentanol, tert.-Pentanol, Hexanole, Heptanole, Glykole, Diglykol, Triglykol.

Der Gegenstand der Erfindung umfasst weiterhin eine Variante des Verfahrens wie vorstehend beschrieben, die sich dadurch auszeichnet, dass Schritt a) und/oder Schritt d) unter Einstrahlung von Mikrowellenstrahlung und/oder unter Beschallung mit Ultraschall durchgeführt wird, etwa indem die Verfahrensschritte in einem Ultraschallbad oder einem Mikrowellenofen durchgeführt werden.

Der Gegenstand der Erfindung umfasst weiterhin eine Variante des Verfahrens wie vorstehend beschrieben, die sich dadurch auszeichnet, dass Schritt d) unter Verwendung einer Pufferlösung durchgeführt wird, so dass durch die Silanisierungsreaktion entstehende Säuren oder Basen neutralisiert werden, wobei die Benetzung der Metalloxidoberfläche wie vorstehend beschrieben erfolgen kann.

Der Gegenstand der Erfindung umfasst weiterhin eine Variante des Verfahrens wie vorstehend beschrieben, die sich dadurch auszeichnet, dass sich an Schritt d) noch mindestens ein weiterer Schritt e) zur Reinigung der gemäß Schritt d) erhaltenen silanisierten Metalloxidoberfläche anschließt, der mindestens eines der Reinigungsverfahren umfasst, ausgewählt aus der Liste, umfassend Waschprozeduren, Trocknen, mechanisches Abwischen, Plasmabehandlung u.a.m.

Der Gegenstand der Erfindung umfasst weiterhin eine Variante des Verfahrens wie vorstehend beschrieben, die sich dadurch auszeichnet, dass es sich bei der Metalloxidoberfläche um eine ZrO₂-Oberfläche oder um eine TiO₂-Oberfläche handelt.

Der Gegenstand der Erfindung umfasst weiterhin eine Variante des Verfahrens wie vorstehend beschrieben, die sich dadurch auszeichnet, dass es sich bei der Metalloxidoberfläche um eine Metalloxidoberfläche handelt, enthaltend mindestens ein Metall oder Halbmetall, ausgewählt aus der Liste umfassend die Metalle bzw. Halbmetalle In, Zr, Ti, Ge, Sn, Pb, Sb, Si. Bezüglich letzterem (Si), dessen Oxid ja bereits - wie weiter oben beschrieben - in größerem Umfange Hydroxylgruppen aufweist als andere Oxide, bewirkt das erfindungsgemäße Verfahren dennoch eine weitere Verbesserung der Silanisierbarkeit/Funktionalisierbarkeit, so daß Si vom Umfang der Erfindung sowie ihrer Äquivalente mit umfasst ist.

Der Gegenstand der Erfindung umfasst weiterhin eine Variante des Verfahrens wie vorstehend beschrieben, die sich dadurch auszeichnet, dass die Metalloxidoberfläche eine Dotierung aufweist, die mindestens ein Element umfasst, ausgewählt aus der Liste umfassend die chemischen Elemente Cu, Ag, Au, Co, Fe, Co, Ni, Ru, Rh, Pd, Os, Ir, Pt, N, S, P, Sc, Y, Lanthanoide (Seltenerdmetalle), Sr, Ba, Rb, Cs, Li.

Der Gegenstand der Erfindung umfasst weiterhin eine Variante des Verfahrens wie vorstehend beschrieben, die sich dadurch auszeichnet, dass es sich bei dem Silanisierungsmittel um ein Silanisierungsmittel handelt, umfassend mindestens eine Verbindung der Formel (**I**),

[(PG)-R₁-Z]ₙ SP [R2-(AG)]m, (**I**)

wobei
AG SiR3R4X ist,
R₁ eine C₁-C₃-Alkylengruppe oder eine substituierte oder unsubstituierte Cyclopropylengruppe ist oder entfällt,
R₂ eine C₁-C₁₀-Alkylengruppe ist oder entfällt,
R₃ eine C₁-C₈-Alkylgruppe, Chlor oder OR₅ ist,
R₄ eine C₁-C₈-Alkylgruppe, Phenyl, Chlor oder OR₅ ist,
R₅ eine C₁-C₆-Alkylgruppe ist,
X OR₅ oder Chlor ist,
Z CO-NR₆ ist, wobei R₆ H oder C₁-C₆-Alkyl ist,
PG eine radikalisch polymerisierbare Gruppe der Formel (**II**), 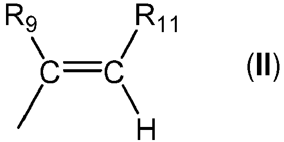
ist, in der R₉ H, C₁-C₃-Alkyl, C₁-C₃-Hydroxyalkyl oder COOR₁₀ ist, wobei R₁₀ H, C₁-C₁₀-Alkyl, 1,6-Dimethylphenyl oder Mesityl ist,
R₁₁ H oder Phenyl ist,
m 1 oder 2 ist,
n 1, 2, 3 oder 4 ist,
SP entfällt oder ein (n+m)-wertiger linearer oder verzweigter aliphatischer C₁-C₃₀-Rest ist, bei dem die Kohlenstoffkette durch O, S, CO-NH, O-CO-NH oder NH-CO-NH unterbrochen sein kann, oder ein (n+m)-wertiger aromatischer C₆-C₁₈-Rest ist, oder ein (n+m)-wertiger cycloaliphatischer C₃-C₁₈-Rest ist, oder ein (n+m)-wertiger heterocyclischer C₃-C₁₈-Rest ist, wobei die Reste unsubstituiert oder durch C₁-C₅-Alkyl, Cl, Br und/oder OH substituiert sein können.

Die Angabe, dass ein Rest durch Fremdatome oder Gruppen, wie Sauerstoff oder Schwefel, unterbrochen sein kann, ist so zu verstehen, dass eines oder mehrere der Fremdatome oder eine oder mehrere der Gruppen in eine Kohlenstoffkette integriert sind. Daraus folgt, dass die Fremdatome oder Gruppen nicht endständig sein können, d.h. eine Anbindung an Nachbargruppen immer über ein Kohlenstoffatom erfolgt, und dass die Zahl der Fremdatome und Gruppen zwangsläufig kleiner als die Zahl der Kohlenstoffatome sein muß. Formel (**I**) erstreckt sich nur auf solche Verbindungen, die mit der chemischen Valenzlehre vereinbar sind. Bevorzugte polymerisationsfähige Gruppen PG sind Vinylgruppen der Formel H₂C=C(-R₉)-, Acrylsäuregruppen der Formel H₂C=C(-COOR₁₀)- sowie Allyl-, Styryl- und/oder Vinylcyclopropyl-Gruppen. Bevorzugte Definitionen der obigen Variablen, die unabhängig voneinander gewählt werden können, sind:
R₁ = eine Methylengruppe, Cyclopropylengruppe oder entfällt,
R₂ = eine C₁-C₃-Alkylengruppe oder entfällt,
R₃ = eine C₁-C₃-Alkylgruppe, Chlor, insbesondere ORs,
R₄ = eine C₁-C₃-Alkylgruppe, Phenyl, Chlor, insbesondere ORs,
R₅ = eine C₁-C₂-Alkylgruppe,
R₆ = H, eine C₁-C₃-Alkylgruppe, insbesondere Methyl,
X = Chlor, insbesondere ORs,
PG = eine Vinylgruppe H₂C=C(R₉)-, in der R₉ = H oder R₉ = CH₃ ist,
m = 1 oder 2,
n = 1 oder 2,
SP = entfällt oder ein (n+m)-wertiger linearer oder verzweigter aliphatischer C₁-C₆-Rest, ein (n+m)-wertiger aromatischer C₆-C₁₀-Rest, ein (n+m)-wertiger cycloaliphatischer C₃-C₁₀-Rest oder ein (n+m)-wertiger heterocyclischer C₃-C₁₀-Rest, wobei die Reste substituiert oder unsubstituiert sein können. Ganz besonders bevorzugt sind Verbindungen, bei denen mindestens zwei und insbesondere alle Variablen eine der bevorzugten Bedeutungen haben.

Der Gegenstand der Erfindung umfasst weiterhin eine Variante des weiter oben beschriebenen Verfahrens, die sich dadurch auszeichnet, dass es sich bei dem Silanisierungsmittel um 3-(Trimethoxysilyl)propylmethacrylat handelt.

Der Gegenstand der Erfindung umfasst weiterhin silanisierte Metalloxidoberflächen, hergestellt nach einem Verfahren wie vorstehend beschrieben.

Der Gegenstand der Erfindung umfasst weiterhin die Verwendung von erfindungsgemäß silanisierten Metalloxidoberflächen, bzw. von erfindungsgemäßen Metalloxidoberflächen, als haftvermittelnde Schichten zur Einpolymerisierung von Gegenständen in Polymere.

Der Gegenstand der Erfindung umfasst weiterhin die Verwendung von erfindungsgemäß silanisierten Metalloxidoberflächen, bzw. von erfindungsgemäßen Metalloxidoberflächen, als haftvermittelnde Schichten zur Einpolymerisierung von Gegenständen in Dentalkomposite.

### Ausführungsbeispiele

Die Herkunft der Metalloxid-Nanopartikel ist grundsätzlich unerheblich. Beliebige kommerziell erhältliche Partikel können mit dem erfindungsgemäßen Verfahren silanisiert, bzw. allgemeiner: oberflächenmodifiziert, werden. Besonders geeignet sind beispielsweise Partikel, die - herstellungsbedingt - noch hydrolysierbare Oberflächen-liganden aufweisen und/oder leicht in polar-protischen Lösungsmitteln dispergierbar sind. Sie können vorteilhafterweise zum Beispiel via Sol-Gel-Verfahren hergestellt worden sein. Beispielhaft wird nachfolgend die mikrowellenunterstützte Herstellung ganz besonders geeigneter Metalloxid-Nanopartikel beschrieben sowie anschließend deren erfindungsgemäße Silanisierung und nachfolgend deren vorteilhafte Verwendung als Polymerfüllstoff als ein möglicher Nachweis für die Vorteilhaftigkeit des erfindungsgemäßen Silanisierungsverfahrens.

Das nachfolgend zunächst aufgeführte Ausführungsbeispiel für die Herstellung von oberflächenfunktionalisierten Titandioxid-Nanopartikeln kann durch geringfügige Anpassungen, die dem Fachmann aus seiner täglichen Laborpraxis geläufig sind, auch für die Herstellung von anderen oberflächen-funktionalisierten Nanopartikeln, beispielsweise Zirconiumdioxid-Nanopartikeln, verwendet werden.

### Beispielhafte Herstellungsvorschrift für Metalloxid-Nanopartikel

Die hier beispielhaft beschriebene Herstellung von zu silanisierenden Titandioxid-Nanopartikeln wurde im Wesentlichen der Offenlegungsschrift EP 3 009 402 A1 entnommen, deren Inhalt beispielhaft als technologischer Hintergrund des erfindungsgemäßen Verfahrens angesehen wird, da derart hergestellte Partikel sich besonders gut erfindungsgemäß silanisieren lassen, weil sie a) bereits sehr gut dispergiert, bzw. leicht redispergierbar, sind und b) sie auch nur geringe Mengen hydrolysierbare Gruppen an der Oberfläche tragen, wie beispielsweise Hexanoat-Gruppen. Dieses Herstellungsverfahren von Metalloxid-Nanopartikeln kann durch geringfügige Anpassungen, die dem Fachmann aus seiner täglichen Laborpraxis geläufig sind, auch für die Herstellung von anderen Metalloxid-Nanopartikeln, beispielsweise Zirconiumdioxid-Nanopartikeln, verwendet werden.

In eine 30-mL-Glasphiole werden ∼0,8 mL Titantetrabutoxid (=776,0 mg; 2,28 mmol) vorgelegt und 0,25 mL Titantetrachlorid (432,5 mg; 2,28 mmol) über eine Einwegspritze mit Kanüle hinzugefügt (Gleichung (1)).

(1) Ti(OC₄H₉)₄ + TiCl₄ → 2TiCl₂(OC₄H₉)₂ (Metathesereaktion)

Die erhaltene gelbe Lösung wird anschließend für 3 Min. bei 100 °C mit Hilfe eines Mikrowellenreaktors erhitzt. Nach Druckluftkühlen auf 70 °C werden 12 mL 1-Hexanol hinzugegeben und die Lösung für 1 Min. bei 140 °C erhitzt (Gleichung (2)).

(2) TiCl₂(OC₄H₉)₂ +2 n-C₆H₁₃OH → TiCl₂(OC₆H₁₃)₂ + 2C₄H₉OH (Alkoholyse)

Im letzten Reaktionsschritt zur Herstellung der beispielhaft verwendbaren Metalloxid-Nanopartikel, werden die ca. 13 mL der Reaktionslösung mit 8 mL tert-Amylalkohol vereint und für ca. 3 Min. bei 140 °C erhitzt (Gleichungen (3) und (4)).

(3) TiCl₂(OC₆H₁₃)₂ +2 CH₃ (CH₂)₄ OH → TiCl₂(OC₅H₁₁)₂ + 2 C₆H₁₃OH (Alkoholyse)

(4) TiCl₂ (OC₅H₁₁)₂ + TiCl₂ (OC₃H₁₁)₂ → 2 TiO₂ + 4 CH₃ (CH₂)₄ Cl (Kondensation)

Die Rührgeschwindigkeit beträgt für alle Heizschritte jeweils 300 U./Min. Nach dem Abkühlen wird die erhaltene Reaktionslösung in 100 mL einer Lösung aus n-Pentan und Diethylether (Verhältnis 80 zu 20 Vol.-%) gegeben. Der erhaltene Niederschlag wird zunächst bei 4000 U./Min. für mind. 5 Min. zentrifugiert und anschließend nochmals mit 30 mL Ethanol gewaschen und erneut zentrifugiert.

### Methode 1 (am Beispiel TiO₂):

### Durchführung der erfindungsgemäßen Vorbehandlung der zu silanisierenden Partikel mit dem polar-protischen Lösungsmittel

4,56 mmol TiO₂-Nanopartikel (insgesamt ca. 365 mg TiO₂) werden in 10 mL Wasser gegeben und darin dispergiert. Anschließend wird die wässrige Partikeldispersion in ca. 250 mL Aceton als Fällungsmittel gegeben. Dabei kommt es zur Präzipitation der Partikel, welche im vorliegenden Beispiel anschließend ohne weitere Aufarbeitung abzentrifugiert und weiterverarbeitet werden können.

### Erfindungsgemäße Durchführung der Silanisierungsreaktion

Zunächst werden in einem 250-mL-Rundkolben beispielsweise 0,4 mL (ca. 417,2 mg; 1,68 mmol) 3-(Trimethoxysilyl)propylmethacrylat (etwa Silan A 174, Fa. Sigma-Aldrich) in 55 mL Tetrahydrofuran vorgelegt. Dazu werden ca. 365 mg schwach feuchte TiO₂-Partikel gegeben. Die so erhaltene Ether-Suspension, bzw. Dispersion, wird anschließend bei Raumtemperatur für 1 bis 3 Tage gerührt oder alternativ geschüttelt.

### Methode 2 (am Beispiel ZrO₂, erfindungsgemäß):

Nicht-funktionalisierte ZrO₂-Nanopartikel werden zunächst bei ca. 80 °C getrocknet und anschließend in Wasser dispergiert. Die Entfernung des Wassers erfolgt sodann durch Abdampfen der Dispersion im Trockenschrank bis zur Trockene. Die getrockneten Partikel werden anschließend direkt in der Silansierungsreaktion eingesetzt: Es werden dazu 0,15 mL (156,7 mg, 0,63 mmol) MPS und 18,47 g THF gemischt und zu den noch unfunktionalisierten ZrO₂-Nanopartikeln gegeben. Die Mischung wird für 30 min im Ultraschallbad belassen und anschließend wird die Reaktion des MPS mit den Partikeln in drei Tagen vervollständigt. Dabei wird die Reaktionslösung nicht gerührt oder auf einen Schüttler verbracht.

### Methode 3 (am Beispiel ZrO₂, erfindungsgemäß):

Die Durchführung erfolgt analog zu Methode 2, jedoch werden die Nanopartikel feucht für die Silanisierung eingesetzt.

### Methode 4 (am Beispiel ZrO₂, erfindungsgemäß):

Die Durchführung erfolgt analog zu Methode 3, jedoch werden die Nanopartikel durch dreimaliges Waschen und Zentrifugieren mit absolutem Ethanol als polarprotischem Lösungsmittel vorbehandelt.

### Methode 5 (am Beispiel ZrO₂, erfindungsgemäß):

Die Durchführung erfolgt analog zu Methode 1, jedoch mit Zircondioxid-Nanopartikeln: Die ZrO₂-Nanopartikel werden zunächst zweimal mit Ethanol gewaschen und abzentrifugiert, anschließend in Wasser dispergiert und mit Aceton gefällt und abzentrifugiert. Dabei werden 200 mL Aceton für 45 mL Dispersion benötigt. Anschließend werden die Partikel feucht für die Silanisierung eingesetzt.

### Beispielhafte Verwendung erfindungsgemäß silanisierter Titandioxid-Nanopartikel als Polymer-Additiv

Zur Herstellung eines Kompositmaterials, welches 10-Gew.-% TiO₂-Partikel enthält, werden 2 g Hexan-1,6-dioldiacrylat mit 34 mL einer THF-Partikeldispersion, die 0,75 Gew.-% TiO₂ enthält (entspricht ∼225 mg TiO₂), gemischt und solange geschüttelt oder gerührt, bis eine homogene Lösung entstanden ist. Der Homogenisierungsprozess kann durch Verwendung eines SpeedMixer™ oder durch Einstrahlung von Ultraschall beschleunigt werden.

Anschließend wird mit Hilfe eines Rotationsverdampfers die erhaltene Partikel-Monomer-Lösung unter Lichtausschluss vollständig von Tetrahydrofuran (THF) befreit. Hierbei ist zu beachten, dass die Wasserbadtemperatur 45 °C nicht übersteigt, um eine vorzeitige Polymerisation zu verhindern. Nach der Destillation kann die erhaltene Lösung, bzw. Mischung (Nanopartikel in Monomer, mit ggf. Resten von THF) unter Lichtausschluss im Kühlschrank oder bei Raumtemperatur für längere Zeit gelagert werden. Die Partikel-Monomer-Mischung kann beispielsweise durch Verwendung einer handelsüblichen UV-Lampe, ggf. unter Zusatz von Photoinitiatoren sowie bedarfsweise ggf. Co-Initiatoren, ausgehärtet werden. Dabei hat sich gezeigt, dass beispielsweise durch die gemeinsame Verwendung der Photoinitiatoren Darocur™ 1173 und Irgacure® 819 sowohl die Tiefen- als auch die Oberflächenhärtung verbessert werden kann.

### Abbildungslegenden

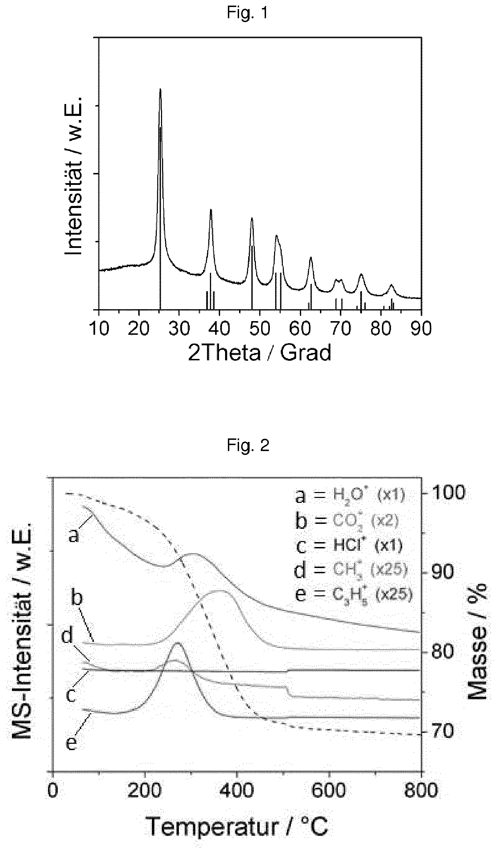

- Fig. 1:: Röntgenpulverdiffraktogramm (Beispiel) von einem erfindungsgemäßen TiO₂-Pulver mit tetragonaler Kristallstruktur (Anatas-Form) nach der Silanisierung mit 3-(Trimethoxysilyl)propylmethacrylat. Die mittlere Korngröße der Anatas-Nanopartikel beträgt in diesem Beispiel (6 ± 1) nm. Das Liniendiagramm unterhalb der Kurve stellt die Beugungsreflexe der JCPDS-Karte Nr. 021-1272 für Anatas dar.
- Fig. 2:: Kombinierte thermogravimetrische und massenspektrometrische Analyse (Beispiel) von einem erfindungsgemäßen TiO₂-Pulver nach der Silanisierung mit 3-(Trimethoxysilyl)propylmethacrylat, gemessen in synthetischer Luft (80% N₂, 20% O₂). Die Angaben in Klammern ("x25", "x1" usw.) geben den Skalierungsfaktor an, mit dem die jeweiligen Werte der betreffenden Kurve in der Graphik zu multiplizieren sind, um die reale Intensität in für die Darstellung geeigneten willkürlichen Einheiten (w. E.) zu erhalten. Die gestrichelte Kurve gibt den GesamtMasseverlust in Prozent an.

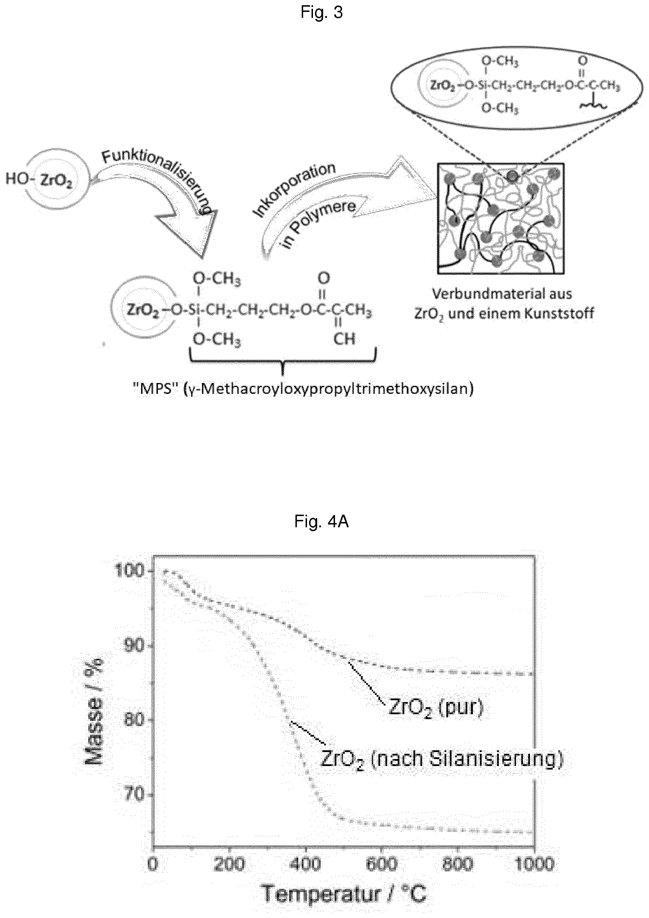

- Fig. 3:: Teilgraphische Übersichtsdarstellung eines Beispiels der erfindungsgemäßen Silanisierung sowie eines Beispiels für die anschließende Verwendung: Partikelfunktionalisierung mit Silan A 174 (3-(Trimethoxysilyl)propylmethacrylat; γ-MPS) sowie chemische Anbindung der funktionalisierten Partikel an ein Polymer.

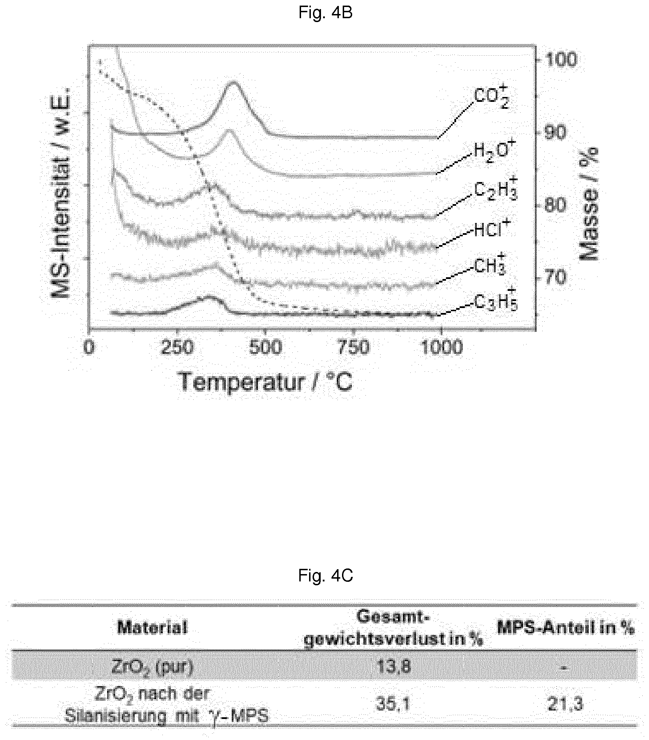

- Fig. 4A-C:: Ergebnisse (Beispiel) einer Thermoanalyse von erfindungsgemäß silanisierten ZrO₂-Nanopartikeln, im Vergleich zu nicht-silanisierten ZrO₂-Nanopartikeln: Kombinierte thermogravimetrische und massenspektrometrische Analyse eines ZrO₂-Pulvers mit und ohne γ-MPS, gemessen in synthetischer Luft (80% N₂, 20% O₂). Fig. 4A: Vergleich des Gesamt-Massenverlustes zwischen silanisierten und nichtsilanisierten Partikeln; Fig. 4B: Ergebnis einer beispielhaften kombinierten Thermoanalyse (Thermogravimetrie + MS-Analyse der Bruchstücke) von silanisierten Partikeln; Fig. 4C: Tabellarische Zusammenstellung der beispielhaften thermogravimetrischen Ergebnisse.
- Fig. 5A-E:: Beispielhafte Analysenergebnisse weiterer Vergleichs- und Ausführungsbeispiele:
Fig. 5A: Kombinierte thermogravimetrische und massenspektrometrische Analyse von unfunktionalisierten ZrO₂-Nanopartikeln in synthetischer Luft (Vergleichsbeispiel) bei einer Heizrate von 5 °C/min. Bei der gestrichelten Linie handelt es sich um die Thermogravimetrie-Kurve. Die MS-Auftragungen zeigen H₂O⁺ (m/z = 18, l/10), ³⁷Cl⁺ (m/z = 37, l · 50), CO₂⁺ (m/z = 44, l · 2), C₄H₈⁺ (m/z = 56, l · 70, Fragmentionsion von C₆H₁₃O) und C₇H₇⁺(m/z = 91, l · 120, Fragmentationsion von C₇H₇O); relative Massenwerte sowie ggf. Intensitätsangaben jeweils in Klammern.
Fig. 5B: Kombinierte thermogravimetrische und massenspektrometrische Analyse von erfindungsgemäß funktionalisierten ZrO₂-Nanopartikeln, hergestellt nach Methode 2, in synthetischer Luft bei einer Heizrate von 5 °C/min. Bei der gestrichelten Linie handelt es sich um die Thermogravimetrie-Kurve. Die MS-Auftragungen zeigen CH₃⁺ (m/z = 15, l · 12), H₂O⁺ (m/z = 18), C₂H₃⁺ (m/z = 27, l · 10), CH₃O⁺ (m/z = 31, l · 30), Cl⁺ (m/z = 35, l · 30), C₃H₅⁺ (m/z = 41, l · 15), C₃H₆⁺ (m/z = 42, l · 20), CO₂⁺ (m/z = 44); relative Massenwerte sowie ggf. Intensitätsangaben jeweils in Klammern.
Fig. 5C: Kombinierte thermogravimetrische und massenspektrometrische Analyse von erfindungsgemäß funktionalisierten ZrO₂-Nanopartikeln, hergestellt nach Methode 3, in synthetischer Luft bei einer Heizrate von 5 °C/min. Bei der gestrichelten Linie handelt es sich um die Thermogravimetrie-Kurve. Die MS-Auftragungen zeigen CH₃⁺ (m/z = 15, l · 15), H₂O⁺ (m/z = 18, l · 2), C₂H₃⁺ (m/z = 27, l · 8), CH₃O⁺ (m/z = 31, l . 30), Cl⁺ (m/z = 35, l · 30), C₃H₅⁺ (m/z = 41, l · 15), C₃H₆⁺ (m/z = 42, l · 15), CO₂⁺(m/z = 44); relative Massenwerte sowie ggf. Intensitätsangaben jeweils in Klammern.
Fig. 5D: Kombinierte thermogravimetrische und massenspektrometrische Analyse von erfindungsgemäß funktionalisierten ZrO₂-Nanopartikeln, hergestellt nach Methode 4, in synthetischer Luft bei einer Heizrate von 5 °C/min. Bei der gestrichelten Linie handelt es sich um die Thermogravimetrie-Kurve. Die MS-Auftragungen zeigen CH₃⁺ (m/z = 15, l · 6), H₂O⁺ (m/z = 18), C₂H₃⁺ (m/z = 27, l · 6), CH₃O⁺ (m/z = 31, l · 6), Cl⁺ (m/z = 35, l · 15), C₃H₅⁺ (m/z = 41, l · 6), C₃H₆⁺ (m/z = 42, l · 6), CO₂⁺ (m/z = 44); relative Massenwerte sowie ggf. Intensitätsangaben jeweils in Klammern.

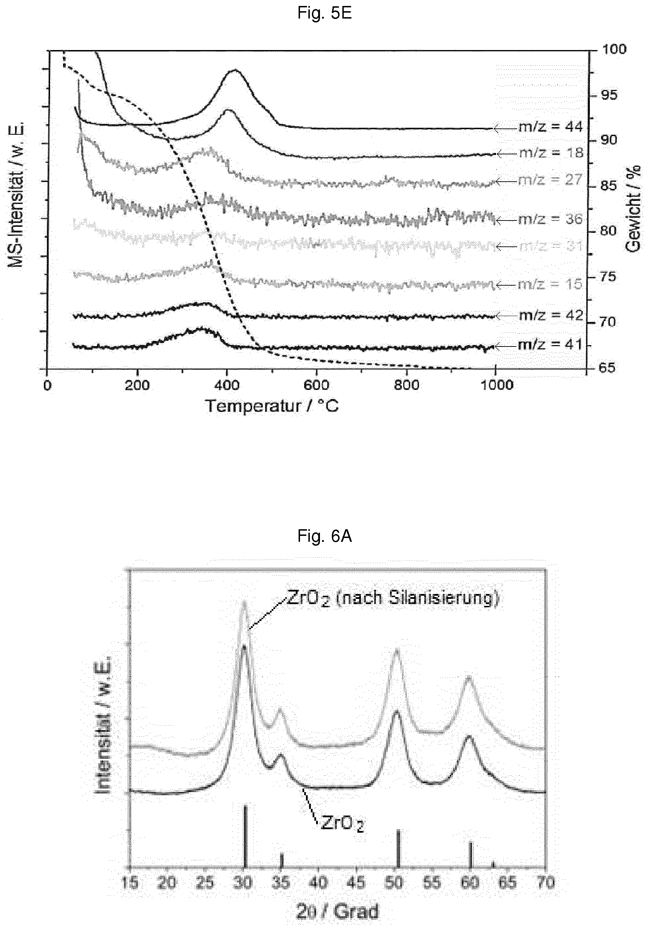

Fig. 5E: Kombinierte thermogravimetrische und massenspektrometrische Analyse von erfindungsgemäß funktionalisierten ZrO₂-Nanopartikeln, hergestellt nach Methode 5, in synthetischer Luft bei einer Heizrate von 5 °C/min. Bei der gestrichelten Linie handelt es sich um die Thermogravimetrie-Kurve. Die MS-Auftragungen zeigen CH₃⁺ (m/z = 15, l · 10), H₂O⁺ (m/z = 18), C₂H₃⁺ (m/z = 27, l · 7), CH₃O⁺ (m/z = 31, l . 15), HCl⁺ (m/z = 36, l . 9), C₃H₅⁺ (m/z = 41, l · 6), C₃H₆⁺ (m/z = 42, l · 6), CO₂⁺ (m/z = 44); relative Massenwerte sowie ggf. Intensitätsangaben jeweils in Klammern.

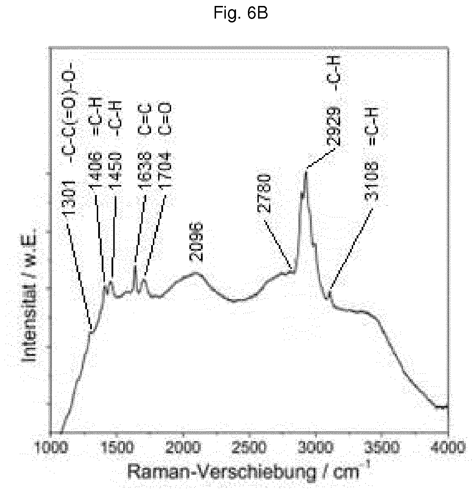

- Fig. 6A-B:: Ergebnisse XRD- und Raman-Daten (Beispiele). Fig. 6A: Röntgenpulverdaten vor und nach der Silanisierung mit γ-MPS; Fig. 6B: RamanSpektrum eines γ-MPS -funktionalisierten ZrO₂-Pulvers.

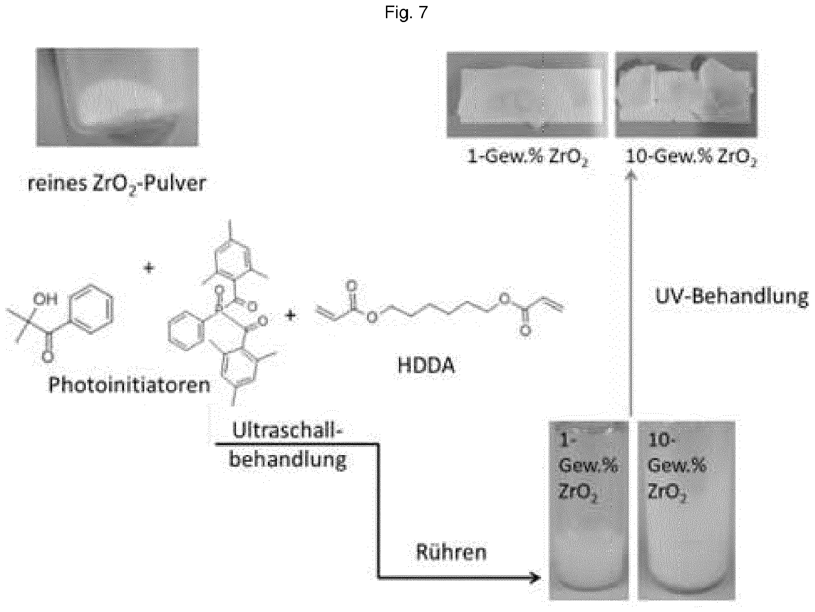

- Fig. 7:: Teilgraphische Übersichtsdarstellung für die beispielhafte Verwendung von Metalloxid-Nanopartikeln als Polymer-Füllstoffe (Vergleichsversuch unter Verwendung nicht-silanisierter Metalloxid-Nanopartikel als Referenz; UV-Strahlenhärtung von 1,6-Hexandioldiacrylat (HDDA) mit ZrO₂-Nanopartikel.). Deutlich ist anhand des trüben Erscheinungsbildes, auch nach UV-Behandlung, die unvollständige Einbindung der Nanopartikel in die Polymermatrix zu erkennen.

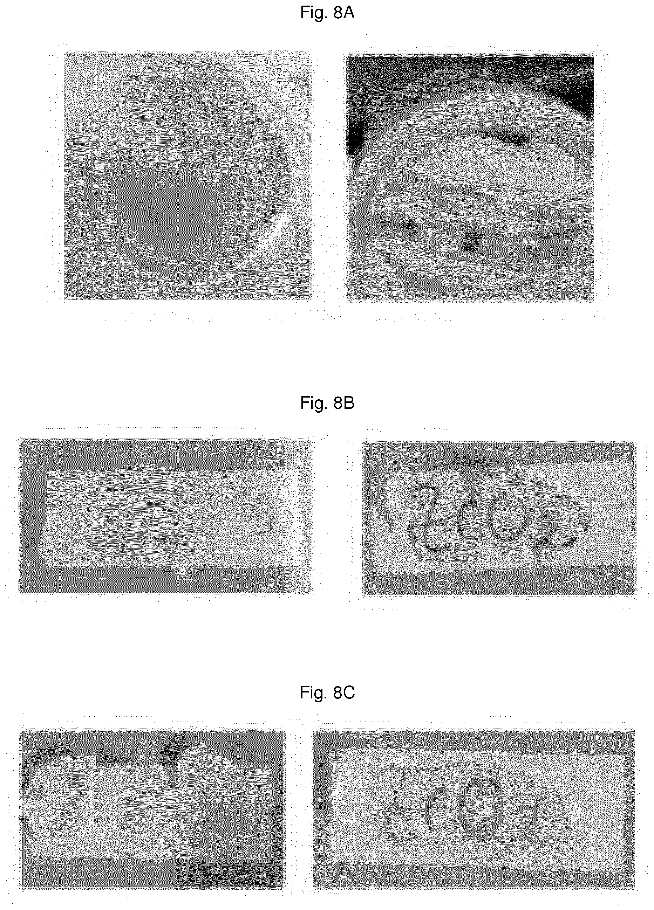

- Fig. 8A-C:: Vergleichende Photodokumentation (Beispiel) für die vorteilhafte Verwendung erfindungsgemäß silanisierter Metalloxid-Nanopartikel als Polymer-Füllstoffe: 1,6-Hexandioldiacrylat (HDDA) nach UVStrahlenhärtung mit ZrO₂-Nanopartikel als Füllstoff. Fig. 8A: UVgehärtetes HDDA-Monomer ohne ZrO₂-Nanopartikel; Fig. 8B: UVgehärtetes HDDA-Monomer mit 1 Gew.-% ZrO₂-Nanopartikeln (links: Kompositmaterial bestehend aus HDDA und nicht-silanisierten (nichtmodifizierten) ZrO₂-Nanopartikeln, rechts: Kompositmaterial bestehend aus HDDA und erfindungsgemäß silanisierten (modifizierten) ZrO₂-Nanopartikeln mit Acrylat-Ankergruppen); Fig. 8C: UV-gehärtetes HDDA-Monomer mit 10 Gew.-% ZrO₂-Nanopartikeln (links: Kompositmaterial bestehend aus HDDA und nicht-silanisierten (nichtmodifizierten) ZrO₂-Nanopartikeln, rechts: Kompositmaterial bestehend aus HDDA und erfindungsgemäß silanisierten (modifizierten) ZrO₂-Nanopartikeln mit Acrylat-Ankergruppen).
- Fig. 9:: Bevorzugte Silanisierungsmittel gemäß Formel (**I**).

## Patentansprüche

1. Verfahren zur Silanisierung von Metalloxid-Nanopartikeln, umfassend die Schritte,
a) Vorbehandlung der Metalloxid-Nanopartikel mit einem polar-protischen Lösungsmittel, umfassend die Dispergierung der Metalloxid-Nanopartikel in dem polar-protischen Lösungsmittel, ausgewählt aus der Liste umfassend Wasser, Methanol, Ethanol, Propanol, Butanol, so dass eine Dispersion entsteht;
b) Fällung der Metalloxid-Nanopartikel aus der in Schritt a) erhaltenen Dispersion entweder
i) durch Zugabe eines polar-aprotischen Fällungsmittels, ausgewählt aus der Liste umfassend Ketone, Aceton, Methylethylketon, Ether, Tetrahydrofuran, 1,4-Dioxan, Trioxan, wobei das polar-aprotische Fällungsmittel mit dem polar-protischen Lösungsmittel aus Schritt a) zumindest teilweise mischbar ist,
oder
ii) durch Zugabe eines polar-protischen Fällungsmittels, das einen größeren Alkylrest aufweist als das in Schritt a) verwendete polar-protische Lösungsmittel, und das mit dem polar-protischen Lösungsmittel aus Schritt a) zumindest teilweise mischbar ist;
c) Abtrennung der in Schritt b) ausgefällten Metalloxid-Nanopartikel von der flüssigen Phase und Überführung in ein bereitgestelltes Lösungsmittel für die Durchführung der Silanisierung im nachfolgenden Schritt d), wobei dieses Lösungsmittel für die Silanisierung ausgewählt ist aus der Liste umfassend Tetrahydrofuran, 1,4-Dioxan;
d) Umsetzung der gemäß Schritt c) abgetrennten Metalloxid-Nanopartikel in dem bereitgestellten Lösungsmittel für die Silanisierung mit einem Silanisierungsmittel, wobei die Zugabe des
Silanisierungsmittels vor und/oder während und/oder nach der Überführung der Metalloxid-Nanopartikel in das bereitgestellte Lösungsmittel gemäß Schritt c) erfolgt;
wobei die Schritte a) bis d) unter Ausschluß von Säuren und Basen bei pH-Werten zwischen pH = 2 bis pH = 10, bevorzugt bei pH-Werten zwischen pH = 4 bis pH = 9, und ganz besonders bevorzugt bei im wesentlichen neutralem pH-Wert im Bereich von etwa pH = 6 bis etwa pH = 8 durchgeführt werden.

2. Verfahren gemäß Anspruch 1, **dadurch gekennzeichnet, dass** die Abtrennung gemäß Schritt c) mindestens eines der Trennungsverfahren umfasst, ausgewählt aus der Liste, umfassend Filtration, Zentrifugation, Sedimentation, Dialyse, Destillation, Trocknung, wobei diese Verfahren einzeln, mehrfach hintereinander, in beliebiger Kombination miteinander sowie mit oder ohne vor-, zwischen- oder nachgeschalteten Waschprozessen vorgenommen werden.

3. Verfahren gemäß einem der vorangehenden Ansprüche, **dadurch gekennzeichnet, dass** das Fällungsmittel gemäß Schritt b) ii) ausgewählt ist aus der Liste umfassend Propanol, iso-Propanol, Butanol, iso-Butanol, tert.-Butanol, Pentanol, iso-Pentanol, tert.-Pentanol, Hexanole, Heptanole, Glykole, Diglykol, Triglykol.

4. Verfahren gemäß einem der vorangehenden Ansprüche, **dadurch gekennzeichnet, dass** Schritt a) und/oder Schritt d) unter Einstrahlung von Mikrowellenstrahlung und/oder unter Beschallung mit Ultraschall durchgeführt wird.

5. Verfahren gemäß einem der vorangehenden Ansprüche, **dadurch gekennzeichnet, dass** Schritt d) in einer Pufferlösung durchgeführt wird, so dass durch die Silanisierungsreaktion entstehende Säuren oder Basen neutralisiert werden.

6. Verfahren gemäß einem der vorangehenden Ansprüche, **dadurch gekennzeichnet, dass** sich an Schritt d) noch mindestens ein weiterer Schritt e) zur Aufarbeitung der gemäß Schritt d) erhaltenen Dispersion von silanisierten Metalloxid-Nanopartikeln anschließt, der mindestens eines der Aufarbeitungsverfahren umfasst, ausgewählt aus der Liste, umfassend Filtration, Zentrifugation, Sedimentation, Dialyse, Destillation, Trocknung.

7. Verfahren gemäß einem der vorangehenden Ansprüche, **dadurch gekennzeichnet, dass** es sich bei den Metalloxid-Nanopartikeln um Metalloxid-Nanopartikel handelt, die vor oder nach der Silanisierung eine mittlere Partikelgröße zwischen 500 nm und 0,5 nm, bevorzugt zwischen 200 nm und 1 nm, besonders bevorzugt zwischen 1,5 nm und 20 nm aufweisen.

8. Verfahren gemäß einem der vorangehenden Ansprüche, **dadurch gekennzeichnet, dass** es sich bei den Metalloxid-Nanopartikeln um ZrO₂-Nanopartikel oder um TiO₂-Nanopartikel handelt.

9. Verfahren gemäß einem der vorangehenden Ansprüche, **dadurch gekennzeichnet, dass** es sich bei den Metalloxid-Nanopartikeln um Metalloxid-Nanopartikel handelt, enthaltend mindestens ein Metall oder Halbmetall, ausgewählt aus der Liste umfassend die Metalle bzw. Halbmetalle In, Zr, Ti, Ge, Sn, Pb, Sb.

10. Verfahren gemäß einem der vorangehenden Ansprüche, **dadurch gekennzeichnet, dass** die Metalloxid-Nanopartikel eine Dotierung aufweisen, die mindestens ein Element umfasst, ausgewählt aus der Liste umfassend die chemischen Elemente Cu, Ag, Au, Co, Fe, Co, Ni, Ru, Rh, Pd, Os, Ir, Pt, N, S, P, Sc, Y, Lanthanoide (Seltenerdmetalle), Sr, Ba, Rb, Cs, Li.

11. Verfahren gemäß einem der vorangehenden Ansprüche, **dadurch gekennzeichnet, dass** es sich bei dem Silanisierungsmittel um ein Silanisierungsmittel handelt, umfassend mindestens eine Verbindung der Formel (**I**),
[(PG)-R₁-Z]ₙSP[R₂-(AG)]ₘ, (**I**)
wobei
AG SiR3R4X ist,
R₁ eine C₁-C₃-Alkylengruppe oder eine substituierte oder unsubstituierte Cyclopropylengruppe ist oder entfällt,
R₂ eine C₁-C₁₀-Alkylengruppe ist oder entfällt,
R₃ eine C₁-C₈-Alkylgruppe, Chlor oder OR₅ ist,
R₄ eine C₁-C₈-Alkylgruppe, Phenyl, Chlor oder OR₅ ist,
R₅ eine C₁-C₆-Alkylgruppe ist,
X OR₅ oder Chlor ist,
Z CO-NR₆ ist, wobei R₆ H oder C₁-C₆-Alkyl ist,
PG eine radikalisch polymerisierbare Gruppe der Formel (**II**), 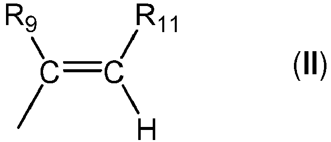 ist, in der
R₉ H, C₁-C₃-Alkyl, C₁-C₃-Hydroxyalkyl oder COOR₁₀ ist, wobei R₁₀ H, C₁-C₁₀-Alkyl, 1,6-Dimethylphenyl oder Mesityl ist,
R₁₁ H oder Phenyl ist,
m 1 oder 2 ist,
n 1, 2, 3 oder 4 ist,
SP entfällt oder ein (n+m)-wertiger linearer oder verzweigter aliphatischer C₁-C₃₀-Rest ist, bei dem die Kohlenstoffkette durch O, S, CO-NH, O-CO-NH oder NH-CO-NH unterbrochen sein kann, oder ein (n+m)-wertiger aromatischer C₆-C₁₈-Rest ist, oder ein (n+m)-wertiger cycloaliphatischer C₃-C₁₈-Rest ist, oder ein (n+m)-wertiger heterocyclischer C₃-C₁₈-Rest ist,
wobei die Reste unsubstituiert oder durch C₁-C₅-Alkyl, Cl, Br und/oder OH substituiert sein können.

12. Verfahren gemäß einem der vorangehenden Ansprüche 1 bis 10, **dadurch gekennzeichnet, dass** es sich bei dem Silanisierungsmittel um 3-(Trimethoxysilyl)propylmethacrylat handelt.

13. Silanisierte Metalloxid-Nanopartikel, hergestellt nach einem Verfahren gemäß einem der vorangehenden Ansprüche 1 bis 12, **dadurch gekennzeichnet, dass** sie bei thermogravimetrischen Messungen bis zu einer Temperatur von 800 °C in synthetischer Luft einen Gesamt-Massenverlust von 10 bis 60 Prozent, bevorzugt von 10 bis 50 Prozent und besonders bevorzugt von 15 bis 40 Prozent, aufweisen.

14. Verwendung von silanisierten Metalloxid-Nanopartikeln gemäß Anspruch 13 als Füllstoff in Polymeren.

15. Verwendung von silanisierten Metalloxid-Nanopartikeln gemäß Anspruch 13 als Füllstoff in Dentalkompositen.
